# EUROPEAN PATENT SPECIFICATION

(11) **EP 4 255 584 B1**
(45) Date of publication and mention of the grant of the patent: **21.08.2024**
(21) Application number: 21827821.6
(22) Date of filing: 30.11.2021
(51) Int. Cl.: A61Q 5/12, A61K 8/85, A61K 8/898

(54) **COMPOSITIONS FOR KERATIN FIBERS**
ZUSAMMENSETZUNGEN FÜR KERATINFASERN
COMPOSITION POUR FIBRES DE KÉRATINE

(30) Priority: 01.12.2020 JP 2020199434; 14.01.2021 FR 2100339
(43) Date of publication of application: 11.10.2023
(73) Proprietor: L'OREAL, 75008 Paris (FR)
(72) Inventor: LEE, Isaac Eng Ting, Kawasaki-shi, Kanagawa 2130012 (JP); SHIBUYA, Yoshiki, Kawasaki-shi, Kanagawa 2130012 (JP); TSAO, Yi-Yun, Kawasaki-shi, Kanagawa 2130012 (JP)
(74) Representative: Lavoix
(86) International application number: PCT/JP2021/044600
(87) International publication number: WO 2022/118979

(56) References cited:
- WO-A1-2012/072765
- FR-A1- 2 984 149
- FR-A1- 3 045 375
- JP-A- 2006 124 352
- JP-B2- 4 754 326
- DATABASE GNPD [online] MINTEL; 21 December 2020 (2020-12-21), ANONYMOUS: "Conditioner", XP055861184, retrieved from https://www.gnpd.com/sinatra/recordpage/8279673/ Database accession no. 8279673
- DATABASE GNPD [online] MINTEL; 21 December 2020 (2020-12-21), ANONYMOUS: "Conditioner", XP055861184, retrieved from https://www.gnpd.com/sinatra/recordpage/8279673/ Database accession no. 8279673

## Description

### TECHNICAL FIELD

The present invention relates to a composition for treating, such as conditioning, keratin fibers such as hair.

### BACKGROUND ART

In the field of hair cosmetics, hair conditioning effects are very important properties. A variety of leave-on type and rinse-off type hair care cosmetic products have been used for conditioning hair.

In order to enhance the above conditioning effects, it has been proposed to add hydrophobic ingredients such as silicones. Silicones, in particular aminosilicones, can repair hair damage effectively, and therefore, they can provide hair conditioning effects.

JP-B-4754326 discloses a cosmetic composition for hair comprising a hydrogenated castor oil/sebacic acid copolymer and an amino-modified silicone with a high polymerization degree of from 3,000 to 20,000 and/or a non-modified silicone with a high polymerization degree of from 3,000 to 20,000.

However, there remains a need to improve hair conditioning effects.

### DISCLOSURE OF INVENTION

An objective of the present invention is to provide a composition which can provide keratin fibers such as hair with improved conditioning effects, in particular a less heavy feeling to the touch and less stickiness.

The above objective can be achieved by a composition for treating keratin fibers comprising:
(a) at least one oligomer of a triglyceride of hydroxylated fatty acid and of a saturated diacid; and
(b) at least one aminosilicone,
wherein
the (b) aminosilicone has a degree of polymerization of less than 2,000.

The triglyceride of hydroxylated fatty acid may be represented by the following formula (A) 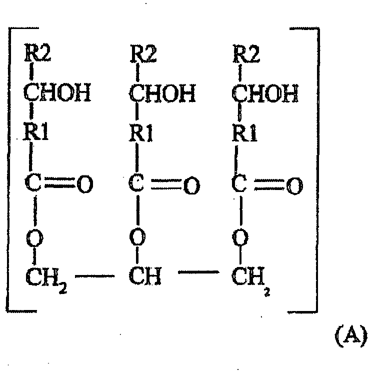 wherein
R₁ represents a saturated or unsaturated, linear or branched alkylene group comprising for example from 1 to 18 carbon atoms, and
R₂ represents a saturated or unsaturated, linear or branched alkyl group comprising for example from 1 to 12 carbon atoms,
   and
the saturated diacid may be represented by the following formula (B) 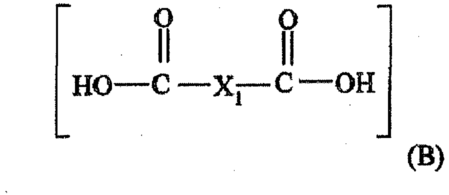 wherein
   Xi is a linear or branched alkylene group, preferably a linear alkylene group -(CH₂)ₓ- where x is an integer from 1 to 30, and preferably from 3 to 15.

The (a) oligomer of a triglyceride of hydroxylated fatty acid and of a saturated diacid may be hydrogenated castor oil/sebacic acid copolymer.

The amount of the (a) oligomer(s) of a triglyceride of hydroxylated fatty acid and of a saturated diacid in the composition according to the present invention may be from 0.1% to 15% by weight, preferably from 0.3% to 10% by weight, and more preferably from 0.5% to 5% by weight, relative to the total weight of the composition.

The amount of the (b) aminosilicone(s) in the composition according to the present invention may be from 0.01% to 15% by weight, preferably from 0.05% to 10% by weight, more preferably from 0.1% to 5% by weight, relative to the total weight of the composition.

The composition according to the present invention may further comprise (c) at least one silicone other than the (b) aminosilicone.

The (c) silicone may have a degree of polymerization of less than 2,000.

The amount of the (c) silicone(s) in the composition according to the present invention may be from 0.01% to 30% by weight, preferably from 0.05% to less than 25% by weight, and more preferably from 0.1% to 20% by weight, relative to the total weight of the composition.

The composition according to the present invention may further comprise (d) at least one ester of polyol(s) and of fatty diacid dimer.

The composition according to the present invention may further comprise (e) water.

The composition according to the present invention may further comprise (f) at least one fatty alcohol.

The composition according to the present invention may further comprise (g) at least one cationic surfactant.

The composition according to the present invention may be a cosmetic composition, preferably a rinse-off type cosmetic composition, and more preferably a rinse-off type hair cosmetic composition.

The present invention also relates to a cosmetic process for caring for or conditioning keratin fibers, preferably hair, comprising the step of:
applying onto the keratin fibers the composition according to the present invention.

The present invention also relates to a use of
(a) at least one oligomer of a triglyceride of hydroxylated fatty acid and of a saturated diacid; and
(b) at least one aminosilicone,
wherein
the (b) aminosilicone has a degree of polymerization of less than 2,000, in a composition for treating keratin fibers in order to enhance or improve the conditioning effects of the composition for the keratin fibers.

### BEST MODE FOR CARRYING OUT THE INVENTION

After diligent research, the inventors have discovered that it is possible to provide a composition which can provide keratin fibers such as hair with improved conditioning effects, in particular a less heavy feeling to the touch and less stickiness.

Thus, the present invention mainly relates to a composition for treating keratin fibers comprising:
(a) at least one oligomer of a triglyceride of hydroxylated fatty acid and of a saturated diacid; and
(b) at least one aminosilicone,
wherein
the (b) aminosilicone has a degree of polymerization of less than 2,000.

The present invention can provide keratin fibers such as hair with improved conditioning effects, in particular a less heavy feeling to the touch and less stickiness.

The composition according to the present invention can also provide keratin fibers with good manageability.

Thus, the present invention is useful for cosmetic treatment of keratin fibers, preferably for conditioning of keratin fibers, and more preferably for providing a light feeling to the touch and smoothness to keratin fibers.

The composition according to the present invention can also provide keratin fibers with a homogeneous coating on the keratin fibers, without providing an excessive heavy/greasy feeling to the touch.

The "keratin fibers" here mean fibers which include at least one keratin substance. It is preferable that at least a part of the surface of the keratin fibers be formed by keratin substances. Examples of keratin fibers include hair, eyebrows, eyelashes, and the like. It is preferable that the present invention be used for treating hair.

Hereafter, the present invention will be described in a detailed manner.

### [Composition]

One aspect of the present invention relates to a composition for treating keratin fibers, such as hair, comprising:
(a) at least one oligomer of a triglyceride of hydroxylated fatty acid and of a saturated diacid; and
(b) at least one aminosilicone,
wherein
the (b) aminosilicone has a degree of polymerization of less than 2,000.

### (Oligomer of Triglyceride of Hydroxylated Fatty Acid and of Saturated Diacid)

The composition according to the present invention comprises (a) at least one oligomer of a triglyceride of hydroxylated fatty acid and of a saturated diacid If two or more oligomers of a triglyceride of hydroxylated fatty acid and of a saturated diacid are used, they may be the same or different.

The term "oligomer" here means a polymer in which a relatively small number of monomers are bonded. It is preferable that the number of monomers in the oligomer be 100 or less, more preferably 50 or less, and even more preferably 20 or less.

The (a) oligomer of a triglyceride of hydroxylated fatty acid and of a saturated diacid can be obtained by the reaction of a triglyceride of a hydroxylated fatty acid (such as hydrogenated castor oil) and of a saturated diacid. The reaction may be esterification. Thus, the (a) oligomer of a triglyceride of hydroxylated fatty acid and of a saturated diacid may be an oligoester of a hydroxylated fatty acid and of a saturated diacid.

According to the present invention, the diacid is said to be saturated when the hydrocarbon-based chain from which it is constituted does not have an unsaturated group, i.e. a carbon-carbon double bond. The term "diacid" here means a hydrocarbon-based compound comprising two -COOH carboxyl functions. The diacid can be a single diacid or a mixture of several different diacids.

Likewise, in the meaning of the present invention, the oligomer can be a mixture of several different oligomers.

Among the saturated diacids that can be used, mention may be made of sebacic acid (or 1,10-decanedioic acid), malonic acid, succinic acid, glutaric acid, adipic acid, azelaic acid, octadecamethylene dicarboxylic acid, and eicosadicarboxylic acid.

More particularly, the oligomer can be an oligoester whose monomers are represented by the following formulae (A) of triglyceride and (B) of diacid: 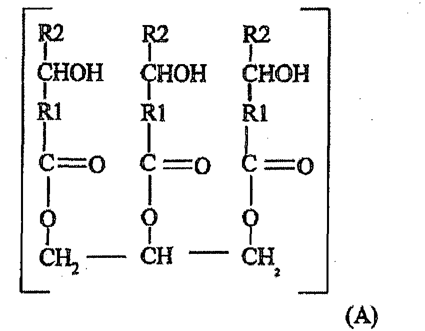 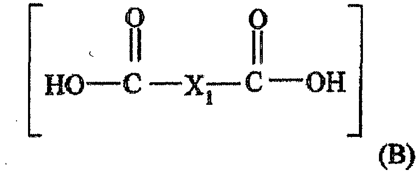 wherein
R₁ represents a saturated or unsaturated, linear or branched alkylene group comprising, for example, from 1 to 18 carbon atoms, preferably represents the group -(CH₂)ₗ-, where "l" can vary from 1 to 20 and especially from 3 to 16, for example from 6 to 12,
R₂ represents a saturated or unsaturated, linear or branched alkyl group comprising, for example, from 1 to 12 carbon atoms, preferably represents the group -(CH₂)ₘ-CH₃, where "m" can vary from 0 to 11 and especially from 2 to 11, for example from 3 to 9,
   and
wherein
X₁ represents a linear or branched alkylene group, preferably represents the linear alkylene group -(CH₂)ₓ- where "x" is an integer from 1 to 30, and preferably from 3 to 15.

According to one embodiment, l=10 and m=5, and the group 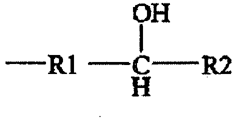 in the above formula (A) represents the alkyl residue of 12-hydroxystearic acid (the major component of hydrogenated castor oil).

When the diacid is sebacic acid, X₁ represents the linear alkylene group -(CH₂)ₓ- where "x" is 8.

The average degree of polymerization of the oligomer may vary between 3 and 12, preferably between 4 and 10.

Thus, the (a) oligomer of a triglyceride of a hydroxylated fatty acid and of a saturated diacid can be represented by the following formula (C): 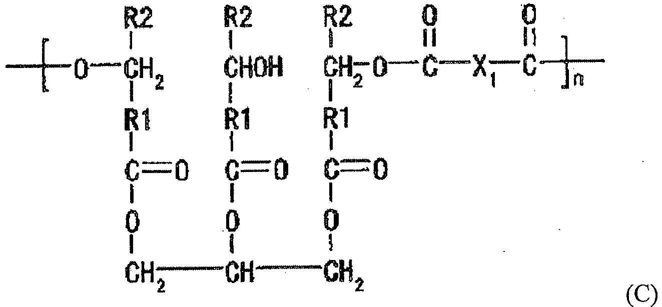 wherein
each of R₁, R₂, and X₁ have the meaning as explained above, and
n means an average degree of polymerization between 3 and 12, preferably between 4 and 10.

It is preferable that the following group 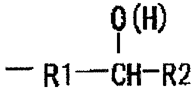 in the above formula (C) represent the alkyl residue of 12-hydroxystearic acid, and that Xi represent -(CH₂)₈-.

It is more preferable that the (a) oligomer of a triglyceride of a hydroxylated fatty acid and of a saturated diacid be an oligoester of hydrogenated castor oil and of sebacic acid, i.e., a hydrogenated castor oil/sebacic acid copolymer formed from hydrogenated castor oil and sebacic acid.

The hydrogenated castor oil/sebacic acid copolymer is especially sold by the company CRODA under various names depending on the degree of polymerization.

Among the hydrogenated castor oil/sebacic acid copolymers, one with a degree of polymerization of about 4.6 is available under the trade name "CROMADOL CWS-5" and one with a degree of polymerization of about 9.5 is available under the trade name "CROMADOL CWS-10", sold by Croda Japan K.K.

Mention may also be made of the oligomer of hydrogenated castor oil and sebacic acid sold under the name CRODABOND CSA (MW=3,500) by the company CRODA.

The (a) oligomer(s) of a triglyceride of a hydroxylated fatty acid and of a saturated diacid may be present in the composition according to the present invention in an amount of 0.1 % by weight or more, preferably 0.3% by weight or more, and more preferably 0.5% by weight or more, relative to the total weight of the composition.

The (a) oligomer(s) of a triglyceride of a hydroxylated fatty acid and of a saturated diacid may be present in the composition according to the present invention in an amount of 15% by weight or less, preferably 10% by weight or less, and more preferably 5% by weight or less, relative to the total weight of the composition.

The (a) oligomer(s) of a triglyceride of a hydroxylated fatty acid and of a saturated diacid may be present in the composition according to the present invention in an amount ranging from 0.1% to 15% by weight, preferably from 0.3% to 10% by weight, and more preferably from 0.5% to 5% by weight, relative to the total weight of the composition.

### (Aminosilicone)

The composition according to the present invention comprises (b) at least one aminosilicone. A single type of aminosilicone may be used, or two or more different types of aminosilicones may be used in combination.

According to the present invention, the (b) aminosilicone has a degree of polymerization of less than 2,000.

The degree of polymerization is the number of repeating units which form a polymer. The degree of polymerization here represents an average value of degree of polymerization, preferably a number average value of degree of polymerization.

As the (b) aminosilicone, any aminosilicone compound in the field of cosmetics may be used.

The term "aminosilicone" here means a silicone comprising at least one primary, secondary or tertiary amine group or at least one quaternary ammonium group.

As the (b) aminosilicone that may be used in the present invention, the following can be cited:
(i) Polysiloxanes corresponding to formula (A): 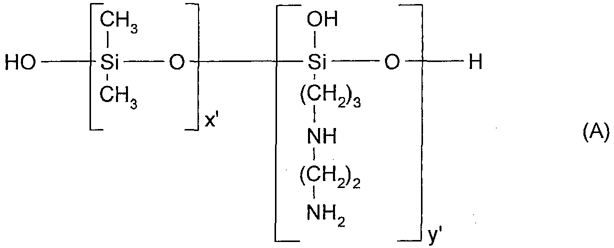 in which x' and y' are independently integers such that the sum of x' and y' is less than 2,000;
(ii) Aminosilicone corresponding to formula (B):

   R'ₐG₍₃₋ₐ₎-Si(OSiG₂)ₙ-(OSiGbR'_{(2-b)})ₘ-O-SiG_{(3-a')}R'_{a'} (B)

   in which:
   G independently designates a hydrogen atom, or a phenyl, OH, or a C₁-C₈ alkyl group, for example methyl, or a C₁-C₈ alkoxy group, for example methoxy,
   a and a' independently denote the number 0 or an integer from 1 to 3, in particular 0;
   b denotes 0 or 1, and in particular 1;
   m and n are numbers such that the sum (n + m) ranges from 1 to less than 2,000 and in particular from 50 to 150, it being possible for n to denote a number from 0 to less than 1,999 and in particular from 49 to 149, and for m to denote a number from 1 to less than 2,000 and in particular from 1 to 10;
   R' independently denotes a monovalent group having formula -C_{q}H_{2q}L in which q is a number ranging from 2 to 8 and L is an optionally quaternized amino group chosen from the following groups:

      -NR"-Q-N(R")₂

      -N(R")₂

      -N⁺(R")₃ A⁻

      -N⁺H(R")₂ A⁻

      -N⁺H₂(R") A⁻

      -NR"-Q-N⁺R"H₂ A⁻

      -NR"-Q-N⁺ (R")₂H A⁻

      -NR"-Q-N⁺ (R")₃ A⁻,
   in which
   R" independently denotes hydrogen, phenyl, benzyl, or a saturated monovalent hydrocarbon-based group, for example a C₁-C₂₀ alkyl group;
   Q denotes a linear or branched CᵣH₂ᵣ group, r being an integer ranging from 2 to 6, preferably from 2 to 4; and
   A⁻ represents a cosmetically acceptable ion, in particular a halide such as fluoride, chloride, bromide or iodide.

A group of aminosilicones corresponding to this definition (B) is represented by the silicones called "trimethylsilylamodimethicone" having formula (C): 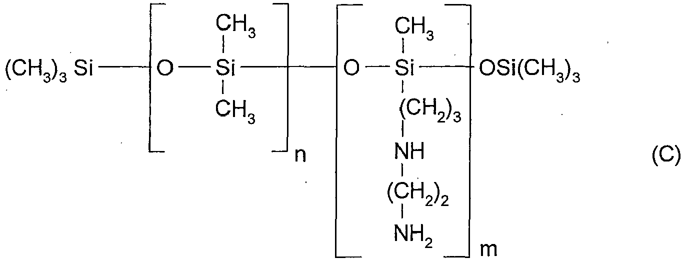 in which n and m have the meanings given above in formula B.

Another group of aminosilicones corresponding to this definition is represented by silicones having the following formula (D) or (E): 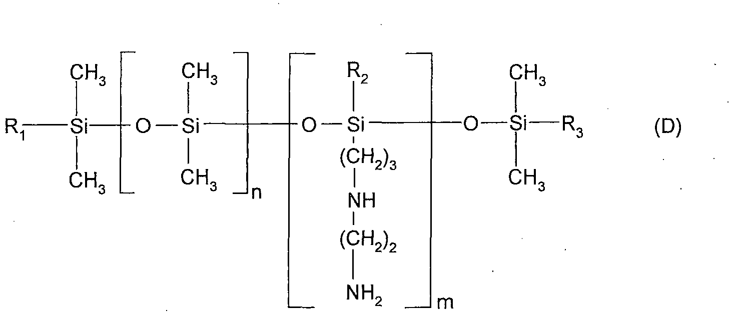 in which:
m and n are numbers such that the sum (n + m) can range from 1 to 1,000, in particular from 50 to 250 and more particularly from 100 to 200, it being possible for n to denote a number from 0 to 999 and in particular from 49 to 249, and more particularly from 125 to 175, and for m to denote a number from 1 to 1,000 and in particular from 1 to 10, and more particularly from 1 to 5;
R₁, R₂, and R₃ independently represent a hydroxy or a C₁-C₄ alkoxy group, wherein at least one of the groups R₁ to R₃ denotes an alkoxy group.
The alkoxy group is preferably a methoxy group.
The hydroxy/alkoxy mole ratio ranges preferably from 0.2:1 to 0.4:1 and preferably from 0.25:1 to 0.35:1 and more particularly equals 0.3:1.

The weight-average molecular weight (Mw) of the silicone ranges preferably from 2,000 to 230,000, more particularly from 3,500 to 150,000. 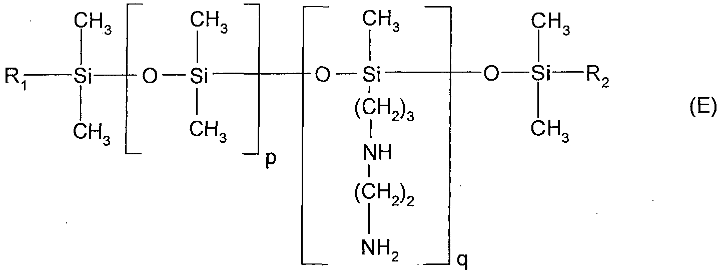 in which:
p and q are numbers such that the sum (p + q) ranges from 1 to 1,000, particularly from 50 to 350, and more particularly from 150 to 250; it being possible for p to denote a number from 0 to 999 and in particular from 49 to 349, and more particularly from 159 to 239 and for q to denote a number from 1 to 1,000, in particular from 1 to 10, and more particularly from 1 to 5;
R₁ and R₂ independently represent a hydroxy or C₁-C₄ alkoxy group, where at least one of the groups R₁ or R₂ denotes an alkoxy group.

The alkoxy group is preferably a methoxy group.

The hydroxy/alkoxy mole ratio ranges generally from 1:0.8 to 1:1.1 and preferably from 1:0.9 to 1:1 and more particularly equals 1:0.95.

The weight-average molecular weight (Mw) of the silicone ranges preferably from 2,000 to 200,000, even more particularly 5,000 to 100,000 and more particularly from 10,000 to 50,000.

Commercial products corresponding to these silicones having structure (D) or (E) may include in their composition one or more other amino silicones whose structure is different from formula (D) or (E).

A product containing aminosilicone having structure (D) is sold by Wacker under the name BELSIL ADM 652.

A product containing aminosilicone having structure (E) is sold by Wacker under the name FLUID WR 1300^{®}.

Another group of aminosilicone corresponding to this definition is represented by the following formula (F): 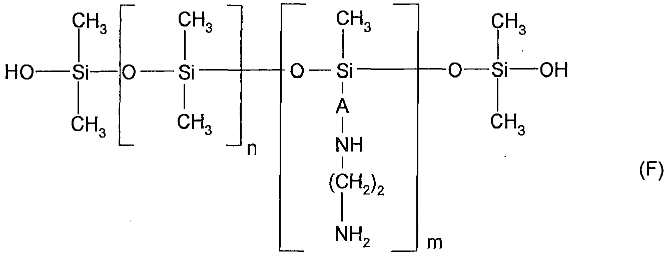 in which:
m and n are numbers such that the sum (n + m) ranges from 1 to less than 2,000 and in particular from 50 to 150, it being possible for n to denote a number from 0 to less than 1,999 and in particular from 49 to 149, and for m to denote a number from 1 to less than 2,000 and in particular from 1 to 10;
A denotes a linear or branched alkylene group containing from 4 to 8 carbon atoms and preferably 4 carbon atoms. This group is preferably linear.

The weight-average molecular weight (Mw) of these aminosilicones ranges preferably from 2,000 to 1,000,000 and even more particularly from 3,500 to 200,000.

A preferred silicone of formula (F) is amodimethicone sold under the trade name XIAMETER^{®} MEM-8299 Cationic Emulsion by Dow Coming.

Another group of aminosilicones corresponding to this definition is represented by the following formula (G): 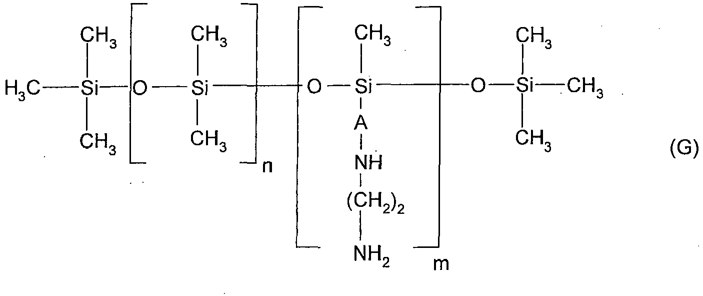 in which:
m and n are numbers such that the sum (n + m) ranges from 1 to less than 2,000 and in particular from 50 to 150, it being possible for n to denote a number from 0 to less than 1,999 and in particular from 49 to 149, and for m to denote a number from 1 to less than 2,000 and in particular from 1 to 10;
A denotes a linear or branched alkylene group containing from 4 to 8 carbon atoms and preferably 4 carbon atoms. This group is preferably branched.

The weight-average molecular weight (Mw) of these aminosilicones ranges preferably from 500 to 1,000,000 and even more particularly from 1,000 to 200,000.

A silicone having this formula is for example DC2-8566 Amino Fluid by Dow Corning.
(iii) Aminosilicone corresponding to formula (H): 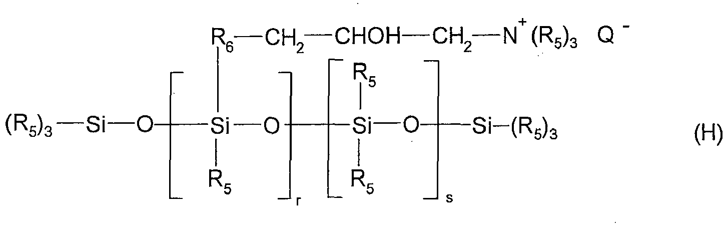 in which:
R₅ independently represents a monovalent hydrocarbon-based group containing from 1 to 18 carbon atoms, and in particular a C₁-C₁₈ alkyl or C₂-C₁₈ alkenyl group, for example methyl;
R₆ represents a divalent hydrocarbon-based group, in particular a C₁-C₁₈ alkylene group or a divalent C₁-C₁₈, for example C₁-C₈, alkyleneoxy group linked to Si via an SiC bond;
Q⁻ is an anion such as a halide ion, in particular chloride, or an organic acid salt (for example acetate);
r represents a mean statistical value from 2 to 20 and in particular from 2 to 8;
s represents a mean statistical value from 20 to 200 and in particular from 20 to 50.

Such aminosilicones are described more particularly in patent US 4 185 087.
(iv) Quaternary ammonium silicones having formula (I): 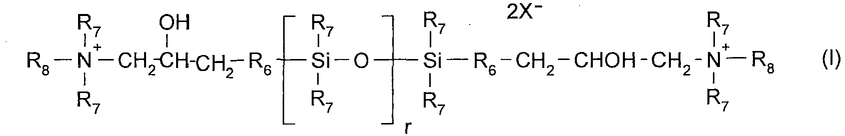 in which:
   R₇ independently represents a monovalent hydrocarbon-based group containing from 1 to 18 carbon atoms, and in particular a C₁-C₁₈ alkyl group, a C₂-C₁₈ alkenyl group or a ring containing 5 or 6 carbon atoms, for example methyl;
   R₆ independently represents a divalent hydrocarbon-based group, in particular a C₁-C₁₈ alkylene group or a divalent C₁-C₁₈, for example C₁-C₈, alkyleneoxy group linked to the Si via a SiC bond;
   R₈ independently represents a hydrogen atom, a monovalent hydrocarbon-based group containing from 1 to 18 carbon atoms, and in particular a C₁-C₁₈ alkyl group, a C₂-C₁₈ alkenyl group or a -R₆-NHCOR₇ group;
   X⁻ is an anion such as a halide ion, in particular chloride, or an organic acid salt (for example acetate);
   r represents a mean statistical value from 2 to 200 and in particular from 5 to 100;
   These silicones are described, for example, in patent application EP-A 0 530 974.
(v) Aminosilicone having formula (J): 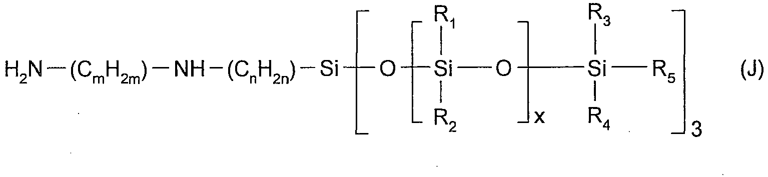 in which:
   R₁, R₂, R₃ and R₄ independently denote a C₁-C₄ alkyl group or a phenyl group;
   R₅ denotes a C₁-C₄ alkyl group or a hydroxy group;
   m is an integer ranging from 1 to 5;
   n is an integer ranging from 1 to 5;
   and in which x is chosen such that the amine number is between 0.01 and 1 meq/g;
(vi) Multiblock polyoxyalkylenated aminosilicone, of type (AB)ₙ, A being a polysiloxane block and B being a polyoxyalkylenated block containing at least one amine group.

Said silicones are preferably constituted of repeating units having the following general formula:

[-(SiMe₂O)ₓSiMe₂ - R -N(R")- R'-O(C₂H₄O)ₐ(C₃H₆O)_{b} -R'-N(H)-R-]

or alternatively

[-(SiMe₂O)ₓSiMe₂ - R -N(R")- R' - O(C₂H₄O)ₐ(C₃H₆O)_{b} -]

in which:
a is an integer greater than or equal to 1, preferably ranging from 5 to 200, more particularly ranging from 10 to 100;
b is an integer comprised between 0 and 200, preferably ranging from 4 to 100, more particularly between from 5 and 30;
x is an integer ranging from 1 to 10,000, more particularly from 10 to 5,000;
R" is a hydrogen atom or a methyl;
R independently represents a divalent linear or branched C₂-C₁₂ hydrocarbon-based group, optionally including one or more heteroatoms such as oxygen; preferably, R independently denotes an ethylene group, a linear or branched propylene group, a linear or branched butylene group, or a -CH₂CH₂CH₂OCH(OH)CH₂- group;
preferentially R independently denotes a -CH₂CH₂CH₂OCH(OH)CH₂- group;
R' independently represent a divalent linear or branched C₂-C₁₂ hydrocarbon-based group, optionally including one or more heteroatoms such as oxygen; preferably, R' denotes an ethylene group, a linear or branched propylene group, a linear or branched butylene group, or a -CH₂CH₂CH₂OCH(OH)CH₂- group; preferentially R' denotes - CH(CH₃)-CH₂-.

The siloxane blocks preferably represent between 50 and 95 mol% of the total weight of the silicone, more particularly from 70 to 85 mol%.

The amine content is preferably between 0.02 and 0.5 meq/g of copolymer in a 30% solution in dipropylene glycol, more particularly between 0.05 and 0.2.

The weight-average molecular weight (Mw) of the silicone is preferably between 5,000 and 1,000,000, more particularly between 10,000 and 200,000.

Mention may be made especially of the silicones sold under the names SILSOFT A-843 or SILSOFT A+ by Momentive.
(vii) Alkylaminosilicone corresponding to formulae (K' and K) below: 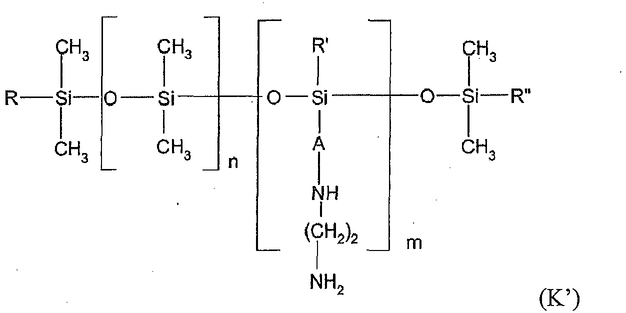 in which
R, R' and R" independently represent a C₁-C₄ alkyl or hydroxy group,
A represents a C₃ alkylene group and m and n are such that the sum of m+n is less than 2,000, preferably less than 1,500, and more preferably less than 1,000 approximately; 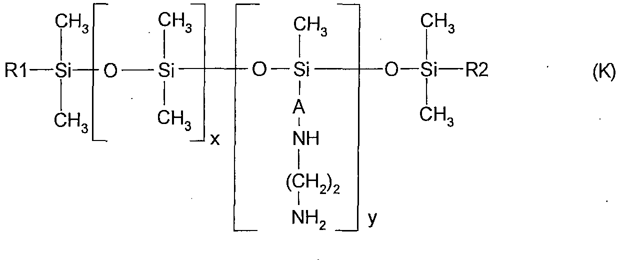 in which:
   x and y are numbers such that the sum of x and y ranges from 1 to less than 2,000;
   preferably, x ranges from 10 to less than 1,500 and especially from 100 to 1,000;
   preferably, y ranges from 1 to 100;
   R1 and R2, preferably independently identical, are linear or branched, saturated or unsaturated alkyl groups, comprising 6 to 30 carbon atoms, preferably 8 to 24 carbon atoms and especially 12 to 20 carbon atoms;
   A denotes a linear or branched alkylene group containing from 2 to 8 carbon atoms. Preferably, A comprises 3 to 6 carbon atoms, especially 4 carbon atoms; preferably, A is branched.
   Mention may be made especially of the following divalent groups: -CH₂CH₂CH₂- and -CH₂CH(CH₃)CH₂-.
   Preferably, R1 and R2 are independently saturated linear alkyl groups comprising 6 to 30 carbon atoms, preferably 8 to 24 carbon atoms and especially 12 to 20 carbon atoms; mention may be made in particular of dodecyl, tetradecyl, pentadecyl, hexadecyl, heptadecyl, octadecyl, nonadecyl and eicosyl groups; and preferentially, R1 and R2, which may be identical or different, are chosen from hexadecyl (cetyl) and octadecyl (stearyl) groups.
   Preferentially, the silicone is of formula (K) with:
      x ranging from 10 to 2,000 and especially from 100 to 1,000;
      y ranging from 1 to 100;
      A comprising 3 to 6 carbon atoms and especially 4 carbon atoms; preferably, A is branched; and more particularly A is chosen from the following divalent groups: CH₂CH₂CH₂ and -CH₂CH(CH₃)CH₂-; and
      R1 and R2 being independently linear, saturated alkyl groups comprising 6 to 30 carbon atoms, preferably 8 to 24 carbon atoms and especially 12 to 20 carbon atoms; chosen in particular from dodecyl, tetradecyl, pentadecyl, hexadecyl, heptadecyl, octadecyl, nonadecyl and eicosyl groups; preferentially, R1 and R2, which may be identical or different, being chosen from hexadecyl (cetyl) and octadecyl (stearyl) groups.
      A preferred silicone of formula (K) is bis-cetearyl amodimethicone.

Mention may be made especially of the silicone sold under the name SILSOFT AX by Momentive.

The aminosilicones of the present disclosure may also be chosen from polydimethylsiloxanes comprising primary amine groups at the chain end or on side chains, for example aminopropyl end or side groups, for instance those of formula (A), (B), or (C):

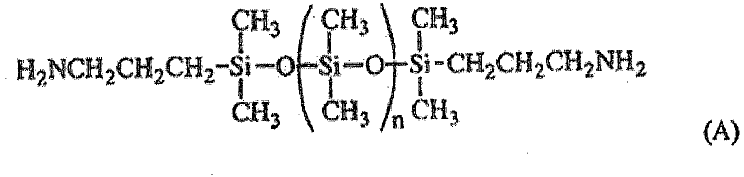

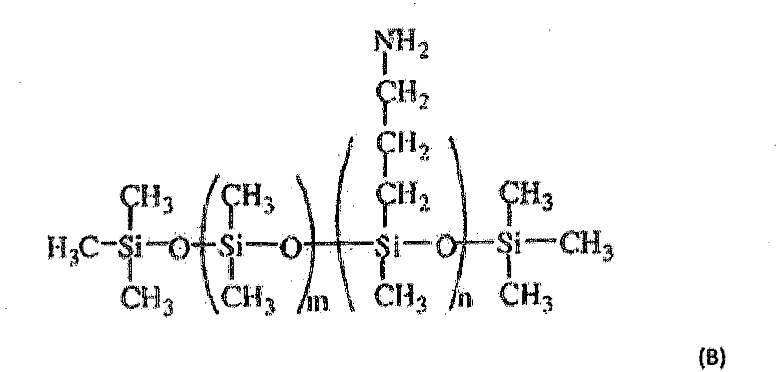

**H₂NCH₂CH₂CHrSi(CH₃)₂-O-[Si(CH₃)₂-O]ₙ-Si(CH₃)₂C₄H₉** **(C)**

In formula (A): the value of n is less than 2,000, preferably less than 1,500, and more preferably less than 1,000. As an example of aminosilicone (A), mention may be made of those sold under the names DMS-A11, DMS-A12, DMS-A15, DMS-A21, DMS-A31, DMS-A32 and DMS-A35 by the company Gelest.

In formula (B), the sum of n and m is such that it is less than 2,000, preferably less than 1,500, and more preferably less than 1,000. As examples of silicone (B), mention may be made of those sold under the names AMS-132, AMS-152, AMS-162, AMS-163, AMS-191 and AMS-1203 by the company Gelest and KF-8015 by the company Shin Etsu.

In formula (C), the value of n is less than 2,000, preferably less than 1,500, and more preferably less than 1,000. As an example of silicone (C), mention may be made of those sold under the names MCR-A11 and MCR-A12 by the company Gelest.

Preferably, the (b) aminosilicone according to the present invention is amodimethicone. In one embodiment, the (b) aminosilicone is bis-cetearyl amodimethicone. In another embodiment, the (b) aminosilicone is aminopropyl dimethicone.

Aminosilicones suitable for use according to the present invention include, but are not limited to, volatile and non-volatile, cyclic, linear, and branched aminosilicones having a viscosity ranging from 5×10⁻⁶ to 2.5 m²/s at 25°C, for example, from 1×10⁻⁵ to 1 m²/s.

The amount of the (b) aminosilicone in the composition according to the present invention may be 0.01% by weight or more, preferably 0.05% by weight or more, and more preferably 0.1% by weight or more, relative to the total weight of the composition.

The amount of the (b) aminosilicone in the composition according to the present invention may be 15% by weight or less, preferably 10% by weight or less, and more preferably 5% by weight or less, relative to the total weight of the composition.

The amount of the (b) aminosilicone in the composition according to the present invention may range from 0.01% to 15% by weight, preferably from 0.05% to 10% by weight, and more preferably from 0.1% to 5% by weight, relative to the total weight of the composition.

### (Silicone)

The composition according to the present invention may further comprise (c) at least one silicone other than the (b) aminosilicone. A single type of silicone may be used, or two or more different types of silicones may be used in combination.

It is preferable that the (c) silicone be selected from silicone oils.

Here, "silicone oil" means a silicone compound or substance which is in the form of a liquid or a paste at room temperature (25°C) under atmospheric pressure (760 mmHg). As the silicone oils, those generally used in cosmetics may be used alone or in combination thereof.

Silicones or organopolysiloxanes are defined, for instance, by Walter NOLL in "Chemistry and Technology of Silicones" (1968), Academic Press. They may be volatile or non-volatile.

Thus, the silicone oil(s) may be selected from volatile silicones, non-volatile silicones and mixtures thereof.

Thus, the silicone oil may comprise either at least one volatile silicone oil or at least one non-volatile silicone oil, or both at least one volatile silicone oil and at least one non-volatile silicone oil.

The volatile or non-volatile silicone may be selected from linear, branched, or cyclic silicones, optionally modified with at least one organo-functional moiety or group.

For example, the silicone oil may be selected from the group consisting of polydialkylsiloxanes such as polydimethylsiloxanes (PDMS), polyalkylarylsiloxanes such as phenyltrimethicone, polydiarylsiloxanes, and organo-modified polysiloxanes comprising at least one organo-functional moiety or group chosen from poly(oxyalkylene) moieties or groups, alkoxy or alkoxyalkyl moieties or groups, hydroxyl or hydroxylated moieties or groups, acyloxy or acyloxyalkyl moieties or groups, carboxylic acid or carboxylate moieties or groups, acrylic moieties or groups, and oxazoline moieties.

If the silicone oil(s) is/are volatile, the silicone oil(s) may be chosen from those having a boiling point ranging from 60°C to 260°C, for example:
(i) cyclic silicones such as polydialkylsiloxanes comprising from 3 to 7, for instance, from 4 to 5 silicon atoms. Non-limiting examples of such siloxanes include octamethylcyclotetrasiloxane marketed, for instance, under the trade name VOLATILE SILICONE^{®} 7207 by UNION CARBIDE and SILBIONE^{®} 70045 V2 by RHODIA, decamethylcyclopentasiloxane marketed under the trade name VOLATILE SILICONE^{®} 7158 by UNION CARBIDE, and SILBIONE^{®} 70045 V5 by RHODIA, KF-995 by SHIN ETSU, as well as mixtures thereof. Cyclomethicones may also be used, for example, those marketed under the references DC 244, DC 245, DC 344, DC 345, and DC 246 by DOW CORNING. Cyclocopolymers of the dimethylsiloxane/methylalkylsiloxane type may also be used, such as SILICONE VOLATILE^{®} FZ 3109 marketed by UNION CARBIDE, of formula 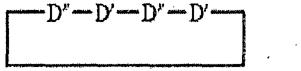 wherein: 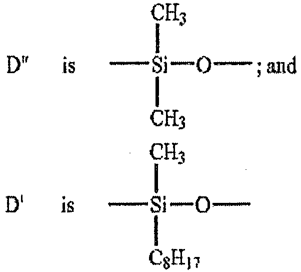 Combinations of cyclic silicones such as polydialkylsiloxanes with silicon derived organic compounds may also be used, such as an octamethylcyclotetrasiloxane and tetratrimethylsilylpentaerythritol (50/50) mixture, and an octamethylcyclotetrasiloxane and oxy-1,1'-(hexa-2,2,2',2',3,3'-irimethylsilyloxy) bis-neopentane mixture; and
(ii) linear volatile polydialkylsiloxanes comprising from 2 to 9 silicon atoms. A non-limiting example of such a compound is decamethyltetrasiloxane marketed, for instance, under the trade name "SH-200" by TORAY SILICONE. Silicones belonging to this class are also described, for example, in Cosmetics and Toiletries, Vol. 91, Jan. 76, P. 27-32--TODD & BYERS "Volatile Silicone Fluids for Cosmetics".

If the silicone oil(s) is/are volatile, the silicone oil(s) may be chosen from cyclic silicones.

On the other hand, the silicone oil(s) may be chosen from non-volatile silicones, such as polydialkylsiloxanes, polyalkylarylsiloxanes, polydiarylsiloxanes, and organo-modified polysiloxanes as explained above.

The molecular weight of the (c) silicone, preferably polydimethylsiloxanes with trimethylsilyl end groups, has a weight-average molecular weight (Mw) of 300,000 or more, preferably 350,000 or more, more preferably 400,000 or more, and preferably 3,000,000 or less, more preferably 2,000,000 or less, even more preferably 1,000,000 or less.

According to one embodiment, the (c) silicone, preferably silicone oil, may be chosen from non-volatile polydialkylsiloxanes, for example, polydimethylsiloxanes with trimethylsilyl end groups known under the trade name dimethicones.

The (c) silicones that are preferred in accordance with the present invention may be the polydimethylsiloxanes with trimethylsilyl end groups, such as the oils having a viscosity at 25°C greater than 1,000,000 cSt (mm²/s), even more preferentially a viscosity greater than 2,000,000 cSt, and even more particularly greater than 5,000,000 cSt, better still greater than 10,000,000 cSt and preferably less than 50,000,000 cSt, better still less than 30,000,000 cSt, even better still less than 15,000,000 cSt.

According to the present invention, all the polydimethylsiloxanes can be used as they are or in the form of solutions, emulsions, nanoemulsions or microemulsions.

Non-limiting examples of commercial products corresponding to such polydialkylsiloxanes include BY22-029 (product of Dow Coming Toray, Co., Ltd.; nonionic emulsion of dimethicone oil), BY22-060 (product of Dow Coming Toray, Co., Ltd.; cationic emulsion containing a solution obtained by diluting highly polymerized dimethicone with a low viscosity silicone), BY22-019 (product of Dow Coming Toray, Co., Ltd.; nonionic and cationic emulsion containing a solution obtained by diluting highly polymerized dimethicone with cyclic silicone), BY22-020 (product of Dow Coming Toray, Co., Ltd.; cationic emulsion containing a solution obtained by diluting a highly polymerized dimethicone with light liquid isoparaffin), KM902 (product of Shin-Etsu Chemical Co., Ltd.; nonionic emulsion of highly polymerized dimethicone), KM903 (product of Shin-Etsu Chemical Co., Ltd.; cationic emulsion containing a solution obtained by diluting a highly polymerized dimethicone with a low viscosity silicone), X-52-2127 (product of Shin-Etsu Chemical Co., Ltd.; cationic emulsion containing a solution obtained by diluting a highly polymerized dimethicone with low viscosity silicone), X-52-2162 (product of Shin-Etsu Chemical Co., Ltd.; nonionic emulsion containing a solution obtained by diluting a highly polymerized dimethicone with low viscosity silicone), EMU101 (product of Momentive Performance Materials, Inc.; nonionic emulsion containing a solution obtained by diluting highly polymerized dimethicone with low viscosity silicone), XS65-B3803 (product of Momentive Performance Materials, Inc.; nonionic emulsion containing a solution obtained by diluting highly polymerized dimethicone with low viscosity silicone), DC 7-3100 (product of Dow Corning Toray Silicone, Co., Ltd.).

Polyalkylarylsiloxanes may be chosen from polydimethyl/methylphenylsiloxanes, linear and/or branched polydimethyl/diphenyl siloxanes.

Non-limiting examples of such polyalkylarylsiloxanes include the products marketed under the following trade names:
SILBIONE^{®} fluids of the 70 641 series from RHODIA; RHODORSIL^{®} fluids of the 70 633 and 763 series from RHODIA;
phenyltrimethicone fluid marketed under the reference DOW CORNING 556 COSMETIC GRADE FLUID by DOW CORNING;
PK series silicones from BAYER, for example, the PK20 product;
PN, PH series silicones from BAYER. for example, the PN1000 and PH1000 products; and some SF series fluids from GENERAL ELECTRIC, such as SF 1023, SF 1154, SF 1250, and SF 1265.

Organo-modified silicones which may be used according to the present invention include, but are not limited to, silicones such as those previously defined and comprising within their structure at least one organo-functional moiety or group linked directly or by means of a hydrocarbon group.

Organo-modified silicones may include, for example, polyorganosiloxanes comprising: polyethyleneoxy and/or polypropyleneoxy moieties optionally comprising C₆-C₁₄ alkyl moieties, such as products called dimethicone copolyols marketed by DOW CORNING under the trade name DC 1248 and under the trade name DC Q2-5220 and SILWET^{®} L 722, L 7500, L 77, and L 711 fluids marketed by UNION CARBIDE and (C₁₂)alkyl-methicone copolyol marketed by DOW CORNING under the trade name Q2 5200;
alkoxylated moieties, such as the product marketed under the trade name "SILICONE COPOLYMER F-755" by SWS SILICONES and ABIL WAX^{®} 2428, 2434, and 2440 by GOLDSCHMIDT;
hydroxylated moieties, such as hydroxyalkyl function-containing polyorganosiloxanes described, for instance, in French Patent Application No. FR-A-85 163 34;
acyloxyalkyl moieties, for example, the polyorganosiloxanes described in U.S. Pat. No. 4,957,732;
anionic moieties of the carboxylic acid type, for example, the products described in European Patent No. 0 186 507, marketed by CHISSO CORPORATION, and carboxylic alkyl anionic moieties, such as those present in the X-22-3701E product marketed by SHIN-ETSU; 2-hydroxyalkyl sulfonate; and 2-hydroxyalkyl thiosulfate such as the products marketed by GOLDSCHMIDT under the trade names <<ABIL^{®} S201» and <<ABIL^{®} S255»;
acrylic moieties, such as the products marketed under the names VS80 and VS70 by 3M; and oxazoline moieties 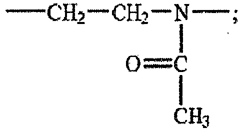
silicones that may be used according to the present invention may comprise 1 or 2 oxazoline groups; for example, poly(2-methyl oxazoline-b-dimethyl siloxane-b-2-methyl oxazoline) and poly(2-ethyl-2-oxazoline-dimethyl siloxane). The products marketed by KAO under the references OX-40, OS-51, OS-96, and OS-88 may also be used.

Polydimethylsiloxanes with dimethylsilanol end groups may also be used, for example, those sold under the trade name dimethiconol (CTFA), such as fluids of the 48 series marketed by RHODIA.

If the silicone oil(s) is/are non-volatile, the silicone oil(s) may be chosen from polydimethylsiloxanes and organo-modified polydimethylsiloxanes.

It is preferable that the silicone oil be selected from volatile or non-volatile silicone oils, such as volatile or non-volatile polydimethylsiloxanes (PDMS) containing a linear or cyclic silicone chain, that are liquid or pasty at ambient temperature, in particular cyclopolydimethylsiloxanes (cyclomethicones) such as cyclopentasiloxane and cyclohexasiloxane; polydimethylsiloxanes containing alkyl, alkoxy, or phenyl groups that are pendent and/or at the end(s) of the silicone chain, which groups have from 2 to 24 carbon atoms; phenyl silicones such as phenyltrimethicones, phenyldimethicones, phenyltrimethylsiloxydiphenylsiloxanes, diphenyldimethicones, diphenylmethyldiphenyltrisiloxanes, 2-phenylethyltrimethyl siloxysilicates, and polymethylphenylsiloxanes; and organo-modified silicones such as dimethiconol.

It is possible to use a combination of at least one volatile silicone and at least one non-volatile silicone, as the silicone oil. Non-limiting examples of such combinations include a mixture of cyclopentasiloxane and dimethiconol, marketed, for instance, under the trade name Xiameter PMX-1501 Fluid by Dow Coming.

It is preferable that the degree of polymerization of the (c) silicone be less than 2,000, more preferably less than 1,500, and even more preferably less than 1,000.

The amount of the (c) silicone in the composition according to the present invention may be 0.01% by weight or more, preferably 0.05% by weight or more, and more preferably 0.1% by weight or more, relative to the total weight of the composition.

The amount of the (c) silicone in the composition according to the present invention may be 30% by weight or less, preferably 25% by weight or less, and more preferably 20% by weight or less, relative to the total weight of the composition.

The amount of the (c) silicone in the composition according to the present invention may range from 0.01% to 30% by weight, preferably from 0.05% to 25% by weight, and more preferably from 0.1% to 20% by weight, relative to the total weight of the composition.

According to one particular embodiment of the present invention, the weight ratio of the (b) aminosilicone/the (c) silicone may be from 0.01 to 5, preferably from 0.1 to 1, and more preferably from 0.2 to 0.5.

### (Ester of Polyol(s) and of Fatty Diacid Dimer or Ester thereof)

The composition according to the present invention may further comprise (d) at least one ester of polyol(s) and of fatty diacid dimer, or an ester thereof. If two or more esters of polyol(s) and of fatty diacid dimer, or ester(s) thereof are used, they may be the same or different.

In the expression "ester of polyol(s) and of fatty diacid dimer, or an ester thereof", the term "or an ester thereof' means one of the derivatives of these esters and of polyol(s) and of fatty diacid dimer obtained either by reaction of alcohol function(s) of the polyol, not engaged in bonds of ester type with acid functions of the diacid dimer, with one or more carboxylic functions of acid molecules other than the diacid dimer, or alternatively by reaction of acid function(s) of the diacid dimer, not engaged in bonds of ester type with alcohol functions of the polyol, with alcohol functions of alcohol molecules other than the polyol.

Advantageously, the esters of polyol(s) and of fatty diacid dimer, or an ester thereof, which are suitable for use in the present invention may have a viscosity, measured at about 25°C of greater than or equal to about 1,500 mPa.s.

The viscosity of an ester of polyol(s) and of fatty diacid dimer, or an ester thereof, according to the present invention may be measured according to any process known to those skilled in the art, and for example according to the conventional process described hereinbelow.

The viscosity may be measured using a cone/plate or parallel plate viscometer of Ares type (TA-Instrument) operating in kinetic sweep mode over a shear range of about 1-1,000 s⁻¹ to induce a flow tension of about 1,000 Pa.

The cone/plate or parallel plates may consist of a material selected from the group consisting of stainless steel, acrylic resins or polyphenylene sulfide (PPS resin).

The cone/plate diameter may be 25 mm (cone angle 0.10 radians).

The measurement is performed at about 25°C.

Before any measurement, the stability of the sample is checked by means of the dynamic sweep period test, which makes it possible to determine if the sample is stable per se.

The shear viscosity is determined using the ETA value in the plateau region according to the flow.

The dynamic sweep period is determined at a frequency of 1.0 Hz over a period of 600 seconds.

The measurements at constant sweep rate are performed with a rate ranging from 1.0 to 1,000 s⁻¹ and for example from 1.0 to 100 s⁻¹.

The viscosity of an ester of polyol(s) and of fatty diacid dimer, or an ester thereof, suitable for use in the present invention may range from about 1,500 mPa.s to about 150,000 mPa.s, or for example from about 2,000 mPa.s to about 150,000 mPa.s, or for example from about 15,000 mPa.s to about 100,000 mPa.s or for example from about 30,000 mPa.s to about 80,000 mPa.s.

According to one embodiment, a polyol that is suitable for use in the present invention may be a diol dimer.

The esters of diol dimer and of fatty diacid dimer that may be used in the context of the present invention are commercially available or may be prepared in a conventional manner. They may be of plant origin and may be obtained by esterification of diacid dimers with diol dimers.

In an esterification reaction with a diacid dimer, a polyol dicarboxylate is obtained, which may have a weight-average molecular weight, determined by gel permeation chromatography (GPC), ranging from 2,000 to 25,000 g/mol or for example between 2,000 and 4,000 g/mol.

### Diacid Dimer:

The fatty diacid dimers, or diacid dimers, may be conventionally obtained by polymerization reaction, for example by intermolecular dimerization, of at least one unsaturated fatty acid.

They may contain at least two carboxylic acid groups.

As indicated previously, the carboxylic functions of the fatty diacid dimer not engaged in the ester bond with the polyol residue(s) may be engaged in other ester bonds with other alcohol functions of alcohol molecules other than the polyol(s).

These alcohol molecules or residues may be monoalcohols or polyols.

As examples of alcohol residues that are suitable for use in the present invention, mention may be made of hydrocarbon-based compounds comprising a hydroxyl function and containing from 4 to 40 carbon atoms, or for example from 6 to 36 carbon atoms, or for example from 8 to 32 carbon atoms, or for example from 16 to 28 carbon atoms, or for example from 18 to 24 carbon atoms.

As examples of monoalcohols that are suitable for the present invention, mention may be made, in a non-limiting manner, of butanol, pentanol, propanol, hexanol, heptanol, octanol, decanol, dodecanol, hexadecanol, octadecanol, eicosadecanol, phytosterol, isostearol, stearol, cetol, behenol, etc.

The fatty diacid dimers may be derived for example from the dimerization of an unsaturated fatty acid, for example of C₈ to C₃₄, or for example of C₁₂ to C₂₂, in particular of C₁₆ to C₂₀ and for example of C₁₈.

As examples of these unsaturated fatty acids, mention may be made for example of undecenoic acid, linderic acid, myristoleic acid, palmitoleic acid, oleic acid, linoleic acid, elaidinic acid, gadolenoic acid, eicosapentaenoic acid, docosahexaenoic acid, erucic acid, brassidic acid and arachidonic acid, and mixtures thereof.

According to one embodiment variant, it may be the diacid dimer from which the diol dimer to be esterified may also be derived. It may be its hydrogenated form.

The hydrogenated form of the diacid dimer may be partial or total, and may correspond, for example, to the saturated form, which is more stable towards oxidation.

According to another embodiment variant, it may be the diacid dimer derived from the dimerization of linoleic acid.

According to one embodiment, the diacid dimer may be a commercial product consisting of a dicarboxylic acid containing about 36 carbon atoms. This product may also contain a trimeric acid and a monomeric acid, in proportions that depend on the degree of purity of the product.

Products whose diacid dimer content may be greater than 70% and others whose diacid dimer content has been adjusted to 90% or more are conventionally found commercially.

Diacid dimers, and for example dilinoleic diacids whose stability towards oxidation has been improved by hydrogenation of the double bonds remaining after the dimerization reaction, may also be found commercially.

In the present invention, any diacid dimer that is currently commercially available may be used.

In an esterification reaction with a diacid dimer, the average degree of esterification and the average molecular weight of the ester obtained may be adjusted by varying the ratio of the diol dimer to the diacid dimer.

### Polyols:

The term "polyol" is intended to denote any hydrocarbon-based compound comprising at least two hydroxyl functions and containing from 4 to 40 carbon atoms, or for example from 6 to 36 carbon atoms, or for example from 8 to 32 carbon atoms, or for example from 16 to 28 carbon atoms and for example from 18 to 24 carbon atoms.

The hydrocarbon-based chains may be interrupted, where appropriate, by the presence of at least one heteroatom, and for example an oxygen atom.

A polyol or a polyol ester that is suitable for use in the present invention may comprise, for example, from 2 to 12 hydroxyl functions, or for example from 2 to 8 hydroxyl functions, or for example from 4 to 6 hydroxyl functions.

Where appropriate, the hydroxyl functions, other than those already employed in an ester bond with the diacid dimer, may also be employed, wholly or partly in other ester bonds via reaction with acid molecules other than the diacid dimer.

The polyol or an ester thereof that is suitable for use in the present invention may be selected from the group consisting of linear, branched, cyclic or polycyclic, saturated or unsaturated alcohols.

Thus, the polyol may be selected, for example, from the group consisting of a diol, a triol, a tetraol, or a pentaol, or an ester thereof.

The polyol may be a diol, or an ester thereof, selected for example from the group consisting of a fatty alcohol dimer, a monoglycerol or polyglycerol, a C₂-C₄ monoalkylene or polyalkylene glycol, 1,4-butanediol and pentaerythritol.

As examples of diols that are also suitable for use in the present invention, mention may be made, in a non-exhaustive manner, of butanediol, pentanediol, propanediol, hexanediol, hexylene glycol, heptanediol, octanediol, nonanediol, decanediol, 1-decanediol, dodecanediol, tridecanediol, tetradecanediol, pentadecanediol, hexadecanediol, nonadecanediol, octadecanediol, cyclohexanediol, diglycerol, erythritol, pentaerythritol, xylitol, sorbitol, ethylene glycol and xylene glycol, and isomers thereof.

According to one variant, as an example of diols that are suitable for use in the present invention, mention may be made of diol dimers.

For the purposes of the present invention, the term "diol dimer" is intended to mean saturated diols derived from the hydrogenation of the corresponding diacid dimers, a diacid dimer being as defined above, and for example a diacid dimer of at least one unsaturated fatty acid.

As regards the industrially manufactured diol dimer, it also generally contains other components, for example a triol trimer, a monoalcohol and compounds of ether type, according to the degree of purification of the acid dimer and/or of the lower alcohol ester thereof, used as starting material.

Generally, products whose diol dimer content is greater than 70% may be used in the present invention. However, one may use a diol dimer of high purity, such as a compound whose diol dimer content is greater than 90%.

Thus, a diol dimer may be produced by hydrogenation, for example catalytic hydrogenation, of a diacid dimer, which is itself obtained by dimerization of at least one unsaturated fatty acid.

A suitable fatty acid may be such as those mentioned previously, and for example of C₈ to C₃₄, or for example of C₁₂ to C₂₂, or for example of C₁₆ to C₂₀ and more particularly of C₁₈.

According to one embodiment, the diol dimer may be derived from the hydrogenation of dilinoleic diacid.

A diol dimer may be, for example, dilinoleol.

The diol dimer may generally be in a saturated form.

As another example of a diol dimer that is suitable for use in the present invention, mention may be made for example of diglycerol.

This compound is a glycerol dimer resulting from the condensation of two molecules of glycerol, with the loss of a water molecule.

The term "diglycerol" denotes any isomer combination that can result from such a condensation, for instance linear isomers, branched isomers and, where appropriate, cyclic isomers resulting from an intramolecular dehydration of a diglycerol molecule.

The diglycerol may be obtained via any process known to those skilled in the art and especially those described in patent EP 0 750 848.

As examples of acid molecules that can interact with one or more hydroxyl functions of the polyol, not engaged in the ester bond with the diacid dimer, mention may be made, in a non-limiting manner, of molecules derived from isostearic acid, behenic acid, phytostearic acid, stearic acid or cetylic acid.

An ester that is suitable for use in the present invention may be obtained by reacting a polyol or an ester thereof with a diacid dimer, and for example a dimerdilinoleic acid, in a molar ratio of about 1.0:0.2-1.

An ester that may be suitable for use in the present invention may be obtained by reacting a dimerdilinoleic acid with a dilinoleol and, where appropriate, at least one additional monoalcohol selected for example from the group consisting of behenol, isostearol, phytosterol, stearol and cetol, and mixtures thereof.

Thus, an ester used in the context of the present invention may be used in the form of a mixture of various esters, for example.

An ester that is suitable for use in the present invention may be obtained, for example, by reacting a glycerol, an isostearic acid and a dimerdilinoleic acid, for example in a molar ratio of 1.0:0.2-1.0:0.5-0.9.

As examples of esters of dimerdilinoleic acid and of polyol(s), or an ester thereof, suitable for the present invention, mention may be made of the esters described in patent applications JP-A-2003-226609, JP-A-2004-256515 and JP-A-2005-179377.

An ester of polyol(s) and of fatty diacid dimer, or an ester thereof, suitable for use in the present invention may have a molecular weight ranging from about 2,000 to about 25,000 g/mol, for example from about 4,000 to about 20,000 g/mol, for example from about 5,000 to about 20,000 g/mol, for example from about 7,000 to about 15,000 g/mol and for example from about 8,000 to about 10,000 g/mol.

According to one embodiment, an ester in accordance with the present invention may comprise an alternating sequence of diacid dimer residue(s) and of residue(s) related to the said polyol(s), and for example to the said diol(s), the said polyols or diols being, for example, as defined above.

Thus, in such a configuration, each of the two ends of the said sequence may bear, respectively, a unit OR' and OR" with R' and R" representing, independently of each other, a hydrogen atom or OR' and OR" representing, independently of each other, a C₂ to C₃₆, for example C₈ to C₂₄, for example C₁₂ to C₂₀ and for example C₁₆ to C₁₈ hydrocarbon based monoalcohol residue.

According to one embodiment, R' and R" may both represent a hydrogen atom.

According to one embodiment, OR' and OR" may both represent an identical or different hydrocarbon-based monoalcohol residue.

As examples of hydrocarbon-based monoalcohol residues OR' and OR" that may be suitable for the present invention, mention may be made of fatty alcohol residues.

An ester that is suitable for use in the present invention may be selected from the group consisting of esters of general formula (I), (II) or (IV) described below, or a mixture thereof.

According to one embodiment, an ester of polyol(s) and of fatty diacid dimer, or an ester thereof, that may be suitable for use in the present invention may have the general formula (I) below:

R₃-OCO-R₁(-COO-R₂-OCO-R₁)ₙ-COO-R₃ (I)

in which:
CORiCO represents a fatty diacid dimer residue,
OR₂O represents a fatty alcohol dimer residue,
OR₃ represents a hydrocarbon-based monoalcohol residue, and
n is an integer ranging from 1 to 15, for example from 2 to 10 or for example from 5 to 7.

According to one embodiment variant, CORiCO may represent a dimerdilinoleate residue.

According to one embodiment variant, OR₂O may represent a dimerdilinoleyl residue.

Moreover, OR₃ may represent a hydrocarbon-based monoalcohol residue selected, for example, from the group consisting of behenyl, isostearyl and phytosteryl residues, and mixtures thereof.

According to another embodiment, the ester of dimerdilinoleic acid and of polyol(s) and of fatty diacid dimer, or an ester thereof, that may be suitable for use in the present invention may for example have the general formula (II) below: 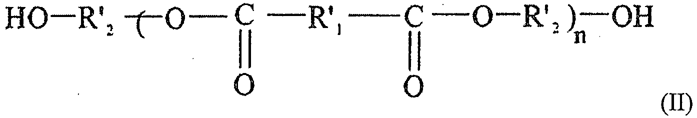 in which:
n is an integer ranging from 1 to 15, for example from 2 to 10 and in particular from 5 to 7,
COR'₁CO represents a fatty diacid dimer residue,
OR'₂O represents a diglyceryl residue of general formula (III) below: 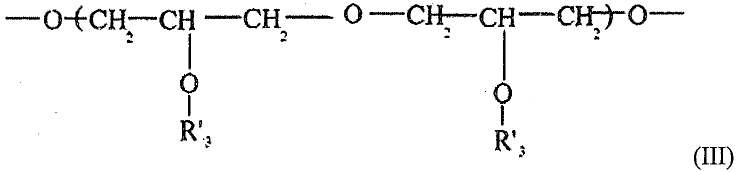 in which:
   R'₃ represents H or OR'₃ represents a fatty acid residue.

According to one embodiment variant, COR'₁CO may represent a dimerdilinoleate residue.

According to one embodiment variant, the fatty acid residue featured by OR'₃ may be an isostearyl residue.

According to one embodiment, an ester of dimerdilinoleic acid and of polyol(s) and of fatty diacid dimer, or an ester thereof, which may be suitable for use in the present invention, may be of formula (IV) below:

HO-R_{1'}-(-OCO-R₂"-COO-R₁"-)ₕ-OH (IV)

in which:
a) OR_{1'}'O represents a diol dimer residue obtained by hydrogenation of a dimerdilinoleic acid,
b) COR₂"CO represents a fatty diacid dimer residue, and
c) h represents an integer ranging from 1 to 9, for example from 2 to 8 and for example from 4 to 6.

According to one embodiment, COR_{2'}'CO may represent a dimerdilinoleate residue.

An ester that is suitable for the present invention may be selected for example from the group consisting of the esters having the following INCI nomenclature: polyglyceryl-2 isostearate dimerdilinoleate copolymer, bis-behenyl/isostearyl/phytosteryl dimerdilinoleyl dimerdilinoleate, dimerdilinoleyl dimerdilinoleate and mixtures thereof.

Such compounds may be obtained, for example, under the reference Hailuscent ISDA (Kokyu Alcohol) and Plandool-G, Plandool-G7, Lusplan DD-DA5, Lusplan DD-DA7, PHY/IS-DA and Lusplan DD-DAS (Nippon Fine Chemical Company Ltd).

For example, Lusplan DD-DA5 and Lusplan DD-DA7 are described in patent application FR 03/02809.

The amount of the (d) ester(s) of polyol(s) and of fatty diacid dimer, or ester(s) thereof, in the composition according to the present invention may be 0.5% by weight or more, preferably 1.0% by weight or more, more preferably 1.5% by weight or more, and even more preferably 2.0% by weight or more, relative to the total weight of the composition.

The amount of the (d) ester(s) of polyol(s) and of fatty diacid dimer, or ester(s) thereof, in the composition according to the present invention may be 20% by weight or less, preferably 15% by weight or less, more preferably 10% by weight or less, and even more preferably 5% by weight or less, relative to the total weight of the composition.

The amount of the (d) ester(s) of polyol(s) and of fatty diacid dimer, or ester(s) thereof, in the composition according to the present invention may range from 0.5% to 20% by weight, preferably from 1.0% to 15% by weight, more preferably from 1.5% to 10% by weight, and even more preferably from 2.0% to 5% by weight, relative to the total weight of the composition.

### (Water)

The composition according to the present invention may further comprise (e) water.

The amount of the (e) water may be 65% by weight or more, preferably 70% by weight or more, and more preferably 75% by weight or more, relative to the total weight of the composition.

The amount of the (e) water may be 95% by weight or less, preferably 90% by weight or less, and more preferably 85% by weight or less, relative to the total weight of the composition.

The amount of the (e) water may be from 65% to 95% by weight, preferably from 70% to 90% by weight, and more preferably from 75% to 85% by weight, relative to the total weight of the composition.

### (Fatty Alcohol)

The composition according to the present invention may further comprise (f) at least one fatty alcohol. Two or more types of fatty alcohols may be used in combination.

The term "fatty" here means the inclusion of a relatively large number of carbon atoms. Thus, alcohols which have 6 or more, preferably 8 or more, and more preferably 10 or more carbon atoms are encompassed within the scope of fatty alcohols. The fatty alcohols may be saturated or unsaturated. The fatty alcohol may be linear or branched. Two or more types of fatty alcohols may be used in combination.

The fatty alcohol may have the structure R-OH wherein R is chosen from saturated and unsaturated, linear and branched radicals containing from 8 to 40 carbon atoms, for example from 8 to 30 carbon atoms. In at least one embodiment, R is chosen from C₁₂-C₂₄ alkyl and C₁₂-C₂₄ alkenyl groups. R may be or may not be substituted with at least one hydroxyl group.

Non-limiting examples of fatty alcohols that may be mentioned include lauryl alcohol, cetyl alcohol, stearyl alcohol, oleyl alcohol, behenyl alcohol, linoleyl alcohol, undecylenyl alcohol, palmitoleyl alcohol, arachidonyl alcohol, erucyl alcohol, cetearyl alcohol, and mixtures thereof.

Examples of suitable fatty alcohols include, but are not limited to, cetyl alcohol, cetearyl alcohol, stearyl alcohol, behenyl alcohol, oleyl alcohol, and mixtures thereof.

The fatty alcohol may represent a mixture of fatty alcohols, which means that several species of fatty alcohol may coexist, in the form of a mixture, in a commercial product.

According to at least one embodiment, the fatty alcohol used in the composition according to the present invention is chosen from cetyl alcohol and cetearyl alcohol.

The amount of the (f) fatty alcohol in the composition according to the present invention may be 0.1% by weight or more, preferably 0.5% by weight or more, and more preferably 1% by weight or more, relative to the total weight of the composition.

The amount of the (f) fatty alcohol in the composition according to the present invention may be 20% by weight or less, preferably 15% by weight or less, and more preferably 10% by weight or less, relative to the total weight of the composition.

The amount of the (f) fatty alcohol in the composition according to the present invention may range from 0.1% to 20% by weight, preferably from 0.5% to 15% by weight, and more preferably from 1% to 10% by weight, relative to the total weight of the composition.

### (Cationic Surfactant)

The composition according to the present invention may further comprise (g) at least one cationic surfactant. Two or more types of cationic surfactants may be used in combination.

The cationic surfactant may be selected from the group consisting of optionally polyoxyalkylenated, primary, secondary or tertiary fatty amine salts, quaternary ammonium salts, and mixtures thereof.

Examples of quaternary ammonium salts that may be mentioned include, but are not limited to:
those of general formula (V) below: 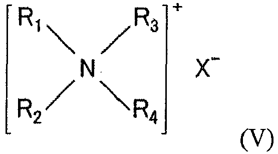
wherein
R₁, R₂, R₃, and R₄, which may be identical or different, are chosen from linear and branched aliphatic groups comprising from 1 to 30 carbon atoms and optionally comprising heteroatoms such as oxygen, nitrogen, sulfur and halogens. The aliphatic groups may be chosen, for example, from alkyl, alkoxy, C₂-C₆ polyoxyalkylene, alkylamide, (C₁₂-C₂₂)alkylamido(C₂-C₆)alkyl, (C₁₂-C₂₂)alkylacetate and hydroxyalkyl groups; and aromatic groups such as aryl and alkylaryl; preferably alkyl groups; and X⁻ is chosen from halides, phosphates, acetates, lactates, (C₁-C₆) alkyl sulfates and alkyl- or alkylaryl-sulfonates; quaternary ammonium salts of imidazoline, for instance those of formula (VI) below: 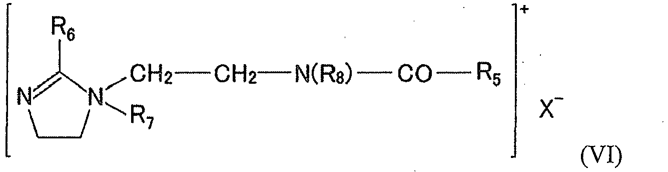 wherein:
   R₅ is chosen from alkenyl and alkyl radicals comprising from 8 to 30 carbon atoms, for example fatty acid derivatives of tallow or of coconut;
   R₆ is chosen from hydrogen, C₁-C₄ alkyl radicals, and alkenyl and alkyl radicals comprising from 8 to 30 carbon atoms;
   R₇ is chosen from C₁-C₄ alkyl radicals;
   R₈ is chosen from hydrogen and C₁-C₄ alkyl radicals; and
   X⁻ is chosen from halides, phosphates, acetates, lactates, alkyl sulfates, alkyl sulfonates, and
   alkylaryl sulfonates. In one embodiment, R₅ and R₆ are, for example, a mixture of radicals chosen from alkenyl and alkyl radicals comprising from 12 to 21 carbon atoms, such as fatty acid derivatives of tallow, R₇ is methyl and R₈ is hydrogen. Examples of such products include, but are not limited to, Quaternium-27 (CTFA 1997) and Quaternium-83 (CTFA 1997), which are sold under the names "Rewoquat^{®}" W75, W90, W75PG and W75HPG by the company Witco;
   diquaternary ammonium salts of formula (VII): 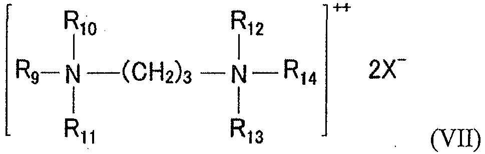 wherein:
      R₉ is chosen from aliphatic radicals comprising from 16 to 30 carbon atoms;
      R₁₀, R₁₁, R₁₂, R₁₃, and R₁₄, which may be identical or different, are chosen from hydrogen and alkyl radicals comprising from 1 to 4 carbon atoms; and
      X⁻ is chosen from halides, acetates, phosphates, nitrates, ethyl sulfates, and methyl sulfates.
      An example of one such diquaternary ammonium salt is propane tallow diammonium dichloride; and
      quaternary ammonium salts comprising at least one ester function, such as those of formula (VIII) below: 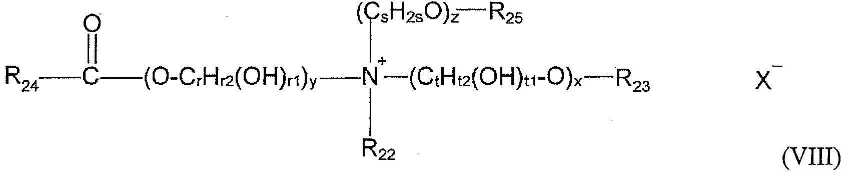 wherein:
         R₂₂ is chosen from C₁-C₆ alkyl radicals, and C₁-C₆ hydroxyalkyl and dihydroxyalkyl radicals;
         R₂₃ is chosen from:
            the radical below: 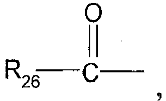
            linear and branched, saturated and unsaturated C₁₋C₂₂ hydrocarbon-based radicals R₂₇, and hydrogen,
            R₂₅ is chosen from:
            the radical below: 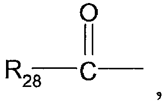
            linear and branched, saturated and unsaturated C₁-C₆ hydrocarbon-based radicals R₂₉, and hydrogen,
            R₂₄, R₂₆, and R₂₈, which may be identical or different, are chosen from linear and branched, saturated and unsaturated, C₇-C₂₁, hydrocarbon-based radicals;
            r, s, and t, which may be identical or different, are chosen from integers ranging from 2 to 6; each of r1 and t1, which may be identical or different, is 0 or 1, and r2+r1=2r and t1+2t=2t;
            y is chosen from integers ranging from 1 to 10;
            x and z, which may be identical or different, are chosen from integers ranging from 0 to 10; X⁻ is chosen from simple and complex, organic and inorganic anions; with the proviso that the sum x+y+z ranges from 1 to 15, that when x is 0, R₂₃ denotes R₂₇, and that when z is 0, R₂₅ denotes R₂₉. R₂₂ may be chosen from linear and branched alkyl radicals. In one embodiment, R₂₂ is chosen from linear alkyl radicals. In another embodiment, R₂₂ is chosen from methyl, ethyl, hydroxyethyl, and dihydroxypropyl radicals, for example methyl and ethyl radicals. In one embodiment, the sum x+y+z ranges from 1 to 10. When R₂₃ is the hydrocarbon-based radical R₂₇, it may be long and comprise from 12 to 22 carbon atoms, or short and comprise from 1 to 3 carbon atoms. When R₂₅ is the hydrocarbon-based radical R₂₉, it may comprise, for example, from 1 to 3 carbon atoms. By way of a non-limiting example, in one embodiment, R₂₄, R₂₆, and R₂₈, which may be identical or different, are chosen from linear and branched, saturated and unsaturated, C₁₁-C₂₁ hydrocarbon-based radicals, for example from linear and branched, saturated and unsaturated C₁₁-C₂₁ alkyl and alkenyl radicals. In another embodiment, x and z, which may be identical or different, are 0 or 1. In one embodiment, y is equal to 1. In another embodiment, r, s, and t, which may be identical or different, are equal to 2 or 3, for example equal to 2. The anion X⁻ may be chosen from, for example, halides, such as chloride, bromide, and iodide; and C₁-C₄ alkyl sulfates, such as methyl sulfate. However, methanesulfonate, phosphate, nitrate, tosylate, an anion derived from an organic acid, such as acetate and lactate, and any other anion that is compatible with the ammonium comprising an ester function, are other non-limiting examples of anions that may be used according to the present invention. In one embodiment, the anion X⁻ is chosen from chloride and methyl sulfate.

In another embodiment, the ammonium salts of formula (VIII) may be used, wherein:
R₂₂ is chosen from methyl and ethyl radicals,
x and y are equal to 1;
z is equal to 0 or 1;
r, s, and t are equal to 2;
R₂₃ is chosen from:
   the radical below: 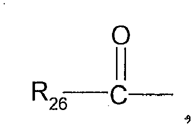
   methyl, ethyl, and C₁₄-C₂₂ hydrocarbon-based radicals, and hydrogen;
   R₂₅ is chosen from:
   the radical below: 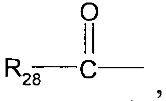
   and hydrogen;
   R₂₄, R₂₆, and R₂₈, which may be identical or different, are chosen from linear and branched, saturated and unsaturated, C₁₃-C₁₇ hydrocarbon-based radicals, for example from linear and branched, saturated and unsaturated, C₁₃-C₁₇ alkyl and alkenyl radicals.

In one embodiment, the hydrocarbon-based radicals are linear.

Non-limiting examples of compounds of formula (VIII) that may be mentioned include salts, for example chloride and methyl sulfate, of diacyloxyethyl-dimethylammonium, of diacyloxyethyl-hydroxyethyl-methylammonium, of monoacyloxyethyl-dihydroxyethyl-methylammonium, of triacyloxyethyl-methylammonium, of monoacyloxyethyl-hydroxyethyl-dimethyl-ammonium, and mixtures thereof. In one embodiment, the acyl radicals may comprise from 14 to 18 carbon atoms, and may be derived, for example, from a plant oil, for instance palm oil and sunflower oil. When the compound comprises several acyl radicals, these radicals may be identical or different.

These products may be obtained, for example, by direct esterification of optionally oxyalkylenated triethanolamine, triisopropanolamine, alkyldiethanolamine, or alkyldiisopropanolamine onto fatty acids or onto mixtures of fatty acids of plant or animal origin, or by transesterification of the methyl esters thereof. This esterification may be followed by a quaternization using an alkylating agent chosen from alkyl halides, for example methyl and ethyl halides; dialkyl sulfates, for example dimethyl and diethyl sulfates; methyl methanesulfonate; methyl para-toluenesulfonate; glycol chlorohydrin; and glycerol chlorohydrin.

Such compounds are sold, for example, under the names Dehyquart^{®} by the company Cognis, Stepanquat^{®} by the company Stepan, Noxamium^{®} by the company Ceca, and "Rewoquat^{®} WE 18" by the company Rewo-Goldschmidt.

Other non-limiting examples of ammonium salts that may be used in the compositions according to the present invention include the ammonium salts comprising at least one ester function described in U.S. Pat. Nos. 4,874,554 and 4,137,180.

Among the cationic surfactants that may be used in the composition according to the present invention, quaternary ammonium and diammonium salts include, for example, distearyldimethylammonium chloride, cetyltimethylammonium chloride (such as, for example, the products sold under the trade name Dehyquart A by Cognis, or Quartamin 60 W25 by Kao, or Genamin CTAC 25 by Clariant), behenyltrimethylammonium chloride (such as the products sold for example by Clariant under the trade name Genamin KDMP or Genamin BTLF, or by Evonik Goldschmidt under the name Varisoft BT 85), behentrimonium chloride, cetrimonium chloride, oleocetyldimethylhydroxyethylammonium chloride, behenoylhydroxypropyltrimethylammonium chloride (such as the product sold by Kao under the name Quartamin BTC 131 ) stearamidopropyldimethyl (myristyl acetate) ammonium chloride, dipalmitoylethylhydroxyethylmethylammonium salt such as dipalmitoylethylhydroxyethylmethylammonium methosulfate (INCI name cetearyl alcohol (and) dipalmitoylethyl hydroxyethylammonium methosulfate) (such as the product Dehyquart F 30 by Cognis), di(CrC₂ alkyl)( C₁₂-C₂₂ alkyl)hydroxy(CrC₂alkyl)ammonium salts, such as dialkyldimethylammonium or alkyltrimethylammonium salt in which the alkyl radical preferably comprises 12 to 24 carbon atoms, propane tallow diammonium dichloride, behentrimonium methosulfate, and mixtures thereof.

Examples of fatty amine salt that may be mentioned include, but are not limited to:
those of general formula (IX) below with a combination with acidifying agents:

RCONHR'N(R")₂ (IX)

wherein
R is a hydrocarbon radical containing at least 6 carbon atoms, preferably from 8 to 30 carbon atoms, more preferably from 12 to 24 carbon atoms. In addition, R can be linear or branched, acyclic or cyclic, saturated or unsaturated, aliphatic or aromatic, substituted or unsubstituted. Typically, R is a linear or branched, acyclic alkyl or alkenyl group or an alkyl phenyl group; and
R' is a divalent hydrocarbon radical containing less than 6 carbon atoms, preferably 2 or 3 carbon atoms, and
R" is H or a hydrocarbon radical containing less than 6 carbon atoms. In addition, R"is linear or branched, acyclic or cyclic, saturated or unsaturated, substituted or unsubstituted. Typically, R" is a linear or branched, acyclic alkyl or alkenyl group. Preferably, R" is H or a methyl group.

In a particular embodiment the cationic surfactant is selected from behentrimonium chloride, cetrimonium chloride, behentrimonium methosulfate, cetrimonium methosulfate, oleamidopropyl dimethylamine, stearamidopropyl dimethylamine, isostearamidopropyl dimethylamine, stearamidoethyl dimethylamine, lauramidopropyl dimethylamine, myristamidopropyl dimethylamine, behenamidopropyl dimethylamine, dilinoleamidopropyl dimethylamine, palmitamidopropyl dimethylamine, ricinoleamindopropyl dimethylamine, soyamidopropyl dimethylamine, wheat germamidopropyl dimethylamine, sunflowerseedamidopropyl dimethylamine, almondamidopropyl dimethylamine, avocadoamidopropyl dimethylamine, babassuamidopropyl dimethylamine, cocamidopropyl dimethylamine, minkamidopropyl dimethylamine, oatamidopropyl dimethylamine, sesamidopropyl dimethylamine, tallamidopropyl dimethylamine, brassicaamidopropyl dimethylamine, olivamidopropyl dimethylamine, palmitamidopropyl dimethylamine, stearamidoethyldiethylamine, and mixtures thereof.

The amount of the (g) cationic surfactant in the composition according to the present invention may be 0.1% by weight or more, preferably 0.5% by weight or more, and more preferably 1% by weight or more, relative to the total weight of the composition.

The amount of the (g) cationic surfactant in the composition according to the present invention may be 15% by weight or less, preferably 10% by weight or less, and more preferably 5% by weight or less, relative to the total weight of the composition.

The amount of the (g) cationic surfactant in the composition according to the present invention may range from 0.1% to 15% by weight, preferably from 0.5% to 10% by weight, and more preferably from 1% to 5% by weight, relative to the total weight of the composition.

### (Optional Components)

The composition according to the present invention may further comprise, in addition to the aforementioned essential/optional components, further optional components typically employed in cosmetics, specifically, nonionic, anionic or amphoteric surfactants, oils, dyes, fillers, polyols such as glycols and glycerol, hydrophilic or lipophilic thickeners, UV filters, natural extracts derived from animals or vegetables, preservatives, and the like, within a range which does not impair the effects of the present invention.

The composition according to the present invention may comprise the above further optional component(s) in an amount of from 0.001% to 30% by weight, preferably from 0.01% to 20% by weight, and more preferably from 0.1% to 10% by weight, relative to the total weight of the composition.

### (Preparation)

The composition according to the present invention can be prepared by mixing the above essential and optional ingredients in accordance with any of the processes which are well known to those skilled in the art.

The composition according to the present invention can be in the form of a fluid, preferably a liquid or a paste, and more preferably a liquid. It is preferable that the composition according to the present invention be in the form of an emulsion.

### (Applications)

The composition according to the present invention can be used for treating, e.g., caring for or conditioning, keratin fibers.

The composition according to the present invention may be a cosmetic composition, preferably a rinse-off type cosmetic composition, and more preferably a rinse-off type hair cosmetic composition

For example, the composition according to the present invention may be used in hair care cosmetic products such as shampoos, conditioners and the like.

### [Cosmetic Process and Use]

The present invention also relates to a cosmetic process for caring for or conditioning keratin fibers, preferably hair, comprising the step of:
applying onto the keratin fibers the composition according to the present invention.

The cosmetic process according to the present invention may further comprise the step of heating the keratin fibers after the step of applying onto the keratin fibers the composition according to the present invention.

The present invention may also relate to a process for providing keratin fibers such as hair with a light feeling to the touch and smoothness, comprising the step of applying onto the keratin fibers the composition according to the present invention.

The applying step can be performed by any conventional means such as an applicator, e.g., a brush.

The present invention also relates to a use of
(a) at least one oligomer of a triglyceride of hydroxylated fatty acid and of a saturated diacid; and
(b) at least one aminosilicone,
wherein
the (b) aminosilicone has a degree of polymerization of less than 2,000, in a composition for treating keratin fibers in order to enhance or improve the conditioning effects of the composition for the keratin fibers. The enhanced or improved conditioning effects include a less heavy feeling to the touch and less stickiness for keratin fibers such as hair.

The explanations for ingredients (a) and (b), as well as other optional ingredients, for the composition according to the present invention can apply to ingredients (a) and (b), as well as possible optional ingredients in the composition for the above use.

The cosmetic process and use according to the present invention can also provide keratin fibers with good manageability.

### EXAMPLES

The present invention will be described in a more detailed manner by way of examples. However, these examples should not be construed as limiting the scope of the present invention.

### Example 1 and Comparative Examples 1 and 2

### [Preparation]

Each of the treatment compositions for hair according to Example 1 (Ex. 1) and Comparative Examples 1 and 2 (Comp. Ex. 1 and Comp. Ex. 2) was prepared by mixing the ingredients shown in Table 1. The numerical values for the amounts of the ingredients are all based on "% by weight" as raw materials.

**Table 1**

| | Ex. 1 | Comp. Ex. 1 | Comp. Ex. 2 |
|---|---|---|---|
| Cetearyl Alcohol (and) Behentrimonium Methosulfate (INCROQUAT BEHENYL TMS-PA-(BR) from Croda) | 10 | 10 | 10 |
| Hydrogenated Castor Oil/Sebacic Acid Copolymer (Crodabond CSA from Croda) | 2 | 2 | 2 |
| Bis-Behenyl/Isostearyl/Phytosteryl Dimer Dilinoleyl Dimer Dilinoleate (Plandool G7, Mw: 8,000, from Nippon Fine Chemicals) | 2 | 2 | 2 |
| Amodimethicone (and) Trideceth-6 (and) Cetrimonium Chloride (Belsil ADM 4000 E from Wacker) | 3 (1.71)* | | |
| Dimethicone (and) Aminopropyl Dimethicone (KF-8017 from Shin-Etsu) | - | 3 (0.3)** | 17.1 (1.71)** |
| Fragrance | 0.5 | 0.5 | 0.5 |
| Phenoxyethanol | 0.9 | 0.9 | 0.9 |
| Water | qsp 100 | qsp 100 | qsp 100 |
| Manageability | 4 | 4 | 5 |
| Heaviness | 4 | 4.5 | 5 |
| Stickiness | 3 | 4 | 5 |

| | | | |
|---|---|---|---|
| * The value in parentheses shows the amount (wt%) of amodimethicone with a lower polymerization degree (less than 2,000). ** The value in parentheses shows the amount (wt%) of aminopropyl dimethicone with a higher polymerization degree (more than 3,000). | | | |

### [Evaluations]

Three hair swatches (hair bundles) (2.7 g, 27 cm) were prepared for each experiment. Each of the hair swatches was washed with a clarifying shampoo once. After shampooing, the hair swatch was rinsed with water. 1.1 g of each of the compositions according to Example 1 and Comparative Examples 1 and 2 was applied onto each hair swatch. The hair swatch was left for 5 minutes. The hair swatch was rinsed off under running water, and then dried naturally under ambient conditions to obtain a treated hair swatch.

The manageability, heaviness, and stickiness, after the above treatment with each of the compositions according to Example 1 and Comparative Examples 1 and 2 onto the hair swatch, were assessed by 3 panelists in accordance with the following criteria. The benchmark means the assessment result for the hair swatch itself, i.e., without the application of any composition.
5: Much higher than benchmark
4: Higher than benchmark
3: Parity to benchmark
2: Lower than benchmark
1: Much lower than benchmark

The scores thus obtained were averaged. The results are shown in Table 1.

### (Results)

As can be seen from the results shown in Table 1, the composition according to Example 1 which comprises an oligomer of a triglyceride of hydroxylated fatty acid and of saturated diacid and an aminosilicone with a low polymerization degree was able to provide improved conditioning effects, in particular a less heavy feeling to the touch and less stickiness. Also, the composition according to Example 1 was able to provide hair with good manageability.

The composition according to Comparative Example 1, which comprises an oligomer of a triglyceride of hydroxylated fatty acid and of saturated diacid and an aminosilicone with a low polymerization degree, provided inferior conditioning effects in terms of a heavier feeling to the touch and more stickiness. The amount of the raw material including aminosilicone in the composition according to Comparative Example 1 was the same as the amount of the raw material including aminosilicone in the composition according to Example 1. On the other hand, the composition according to Comparative Example 1 was able to provide hair with good manageability.

The composition according to Comparative Example 2, which comprises an oligomer of a triglyceride of hydroxylated fatty acid and of saturated diacid and an aminosilicone with a low polymerization degree, provided inferior conditioning effects in terms of a heavier feeling to the touch and more stickiness. The amount of the active ingredient (aminosilicone) in the composition according to Comparative Example 2 was the same as the amount of the active ingredient (aminosilicone) in the composition according to Example 1. On the other hand, the composition according to Comparative Example 2 was able to provide hair with good manageability.

## Claims

1. A composition for treating keratin fibers comprising:
(a) at least one oligomer of a triglyceride of hydroxylated fatty acid and of a saturated diacid; and
(b) at least one aminosilicone,
wherein
the (b) aminosilicone has a degree of polymerization of less than 2,000.

2. The composition according to Claim 1, wherein the triglyceride of hydroxylated fatty acid is represented by the following formula (A) 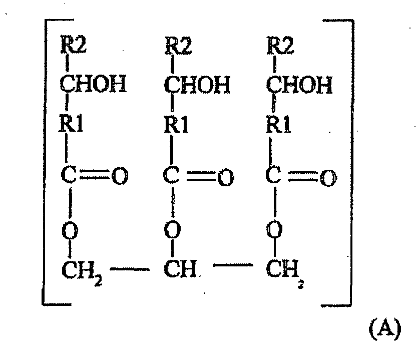 wherein
R₁ represents a saturated or unsaturated, linear or branched alkylene group comprising for example from 1 to 18 carbon atoms, and
R₂ represents a saturated or unsaturated, linear or branched alkyl group comprising for example from 1 to 12 carbon atoms,
and
the saturated diacid is represented by the following formula (B) 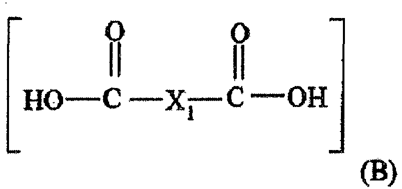 wherein
Xi is a linear or branched alkylene group, preferably a linear alkylene group -(CH₂)ₓ-where x is an integer from 1 to 30, and preferably from 3 to 15.

3. The composition according to Claim 1 or 2, wherein the (a) oligomer of a triglyceride of hydroxylated fatty acid and of a saturated diacid is hydrogenated castor oil/sebacic acid copolymer.

4. The composition according to any one of Claims 1 to 3, wherein the amount of the
(a) oligomer(s) of a triglyceride of hydroxylated fatty acid and of a saturated diacid is from 0.1% to 15% by weight, preferably from 0.3% to 10% by weight, and more preferably from 0.5% to 5% by weight, relative to the total weight of the composition.

5. The composition according to any one of Claims 1 to 4, wherein the amount of the (b) aminosilicone(s) is from 0.01% to 15% by weight, preferably from 0.05% to 10% by weight, more preferably from 0.1% to 5% by weight, relative to the total weight of the composition.

6. The composition according to any one of Claims 1 to 5, wherein the composition further comprises (c) at least one silicone other than the (b) aminosilicone.

7. The composition according to any one of Claims 1 to 6, wherein the (c) silicone has a degree of polymerization of less than 2,000.

8. The composition according to any one of Claims 1 to 7, wherein the amount of the (c) silicone(s) is from 0.1% to 30% by weight, preferably from 0.5% to less than 25% by weight, and more preferably from 1% to 20% by weight, relative to the total weight of the composition.

9. The composition according to any one of Claims 1 to 8, wherein the composition further comprises (d) at least one ester of polyol(s) and of fatty diacid dimer, or an ester thereof.

10. The composition according to any one of Claims 1 to 9, wherein the composition further comprises (e) water.

11. The composition according to any one of Claims 1 to 10, wherein the composition further comprises (f) at least one fatty alcohol.

12. The composition according to any one of Claims 1 to 11, wherein the composition further comprises (g) at least one cationic surfactant.

13. The composition according to any one of Claims 1 to 12, wherein the composition is a cosmetic composition, preferably a rinse-off type cosmetic composition, and more preferably a rinse-off type hair cosmetic composition.

14. A cosmetic process for caring for or conditioning keratin fibers, preferably hair, comprising the step of:
applying onto the keratin fibers the composition according to any one of Claims 1 to 13.

15. A use of
(a) at least one oligomer of a triglyceride of hydroxylated fatty acid and of a saturated diacid; and
(b) at least one aminosilicone,
wherein
the (b) aminosilicone has a degree of polymerization of less than 2,000, in a composition for treating keratin fibers in order to enhance or improve the conditioning effects of the composition for the keratin fibers.

## Patentansprüche

1. Zusammensetzung zum Behandeln von Keratinfasern, umfassend:
(a) mindestens ein Oligomer aus einem Triglycerid einer hydroxylierten Fettsäure und einer gesättigten Disäure; und
(b) mindestens ein Aminosilikon,
wobei
das (b) Aminosilikon einen Polymerisationsgrad von weniger als 2000 aufweist.

2. Zusammensetzung nach Anspruch 1, wobei das Triglycerid der hydroxylierten Fettsäure durch die folgende Formel (A) dargestellt wird 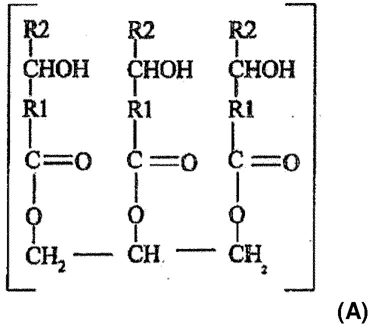 wobei
R₁ eine gesättigte oder ungesättigte, lineare oder verzweigte Alkylengruppe, die beispielsweise 1 bis 18 Kohlenstoffatome umfasst, darstellt und
R₂ eine gesättigte oder ungesättigte, lineare oder verzweigte Alkylgruppe, die beispielsweise 1 bis 12 Kohlenstoffatome umfasst, darstellt
und
die gesättigte Disäure durch die folgende Formel (B) dargestellt wird 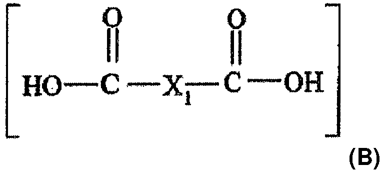
wobei
X₁ eine lineare oder verzweigte Alkylengruppe, vorzugsweise eine lineare Alkylengruppe -(CH₂)_{X}-, wobei x eine ganze Zahl von 1 bis 30 und vorzugsweise von 3 bis 15 ist.

3. Zusammensetzung nach Anspruch 1 oder 2, wobei das (a) Oligomer eines Triglycerids einer hydroxylierten Fettsäure und einer gesättigten Disäure ein hydriertes Rizinusöl/Sebsäure-Copolymer ist.

4. Zusammensetzung nach einem der Ansprüche 1 bis 3, wobei die Menge des (a) Oligomers (der Oligomere) eines Triglycerids einer hydroxylierten Fettsäure und einer gesättigten Disäure 0,1 bis 15 Gew.-%, vorzugsweise 0,3 bis 10 Gew.-% und besonders bevorzugt 0,5 bis 5 Gew.-%, bezogen auf das Gesamtgewicht der Zusammensetzung, beträgt.

5. Zusammensetzung nach einem der Ansprüche 1 bis 4, wobei die Menge des (b) Aminosilikons (der Aminosilikone) von 0,01 bis 15 Gew.-%, vorzugsweise von 0,05 bis 10 Gew.-%, besonders bevorzugt von 0,1 bis 5 Gew.-%, bezogen auf das Gesamtgewicht der Zusammensetzung, beträgt.

6. Zusammensetzung nach einem der Ansprüche 1 bis 5, wobei die Zusammensetzung ferner (c) mindestens ein Silikon umfasst, das von (b) Aminosilikon verschieden ist.

7. Zusammensetzung nach einem der Ansprüche 1 bis 6, wobei das (c) Silikon einen Polymerisationsgrad von weniger als 2000 aufweist.

8. Zusammensetzung nach einem der Ansprüche 1 bis 7, wobei die Menge des (c) Silikons (der Silikone) 0,1 bis 30 Gew.-%, vorzugsweise 0,5 Gew.-% bis weniger als 25 Gew.-% und besonders bevorzugt 1 bis 20 Gew.-%, bezogen auf das Gesamtgewicht der Zusammensetzung, beträgt.

9. Zusammensetzung nach einem der Ansprüche 1 bis 8, wobei die Zusammensetzung ferner (d) mindestens einen Ester aus Polyol(en) und Fettsäuredimeren oder einen Ester davon umfasst.

10. Zusammensetzung nach einem der Ansprüche 1 bis 9, wobei die Zusammensetzung ferner (e) Wasser umfasst.

11. Zusammensetzung nach einem der Ansprüche 1 bis 10, wobei die Zusammensetzung ferner (f) mindestens einen Fettalkohol umfasst.

12. Zusammensetzung nach einem der Ansprüche 1 bis 11, wobei die Zusammensetzung ferner (g) mindestens ein kationisches Tensid umfasst.

13. Zusammensetzung nach einem der Ansprüche 1 bis 12, wobei die Zusammensetzung eine kosmetische Zusammensetzung ist, vorzugsweise eine abspülbare kosmetische Zusammensetzung und noch bevorzugter eine abspülbare kosmetische Zusammensetzung für Haare.

14. Kosmetisches Verfahren zur Pflege oder Konditionierung von Keratinfasern, vorzugsweise Haaren, umfassend den folgenden Schritt:
Auftragen der Zusammensetzung nach einem der Ansprüche 1 bis 13 auf die Keratinfasern.

15. Verwendung von
(a) mindestens einem Oligomer aus einem Triglycerid einer hydroxylierten Fettsäure und einer gesättigten Disäure; und
(b) mindestens einem Aminosilikon,
wobei
das (b) Aminosilikon einen Polymerisationsgrad von weniger als 2000 aufweist, in einer Zusammensetzung zur Behandlung von Keratinfasern, um die konditionierenden Wirkungen der Zusammensetzung für die Keratinfasern zu verstärken oder zu verbessern.

## Revendications

1. Composition pour le traitement de fibres kératiniques comprenant :
(a) au moins un oligomère d'un triglycéride d'acide gras hydroxylé et d'un diacide saturé ; et
(b) au moins un silicone aminé,
dans laquelle
le (b) silicone aminé a un degré de polymérisation inférieur à 2000.

2. Composition selon la revendication 1, dans laquelle le triglycéride d'acide gras hydroxylé est représenté par la formule (A) suivante 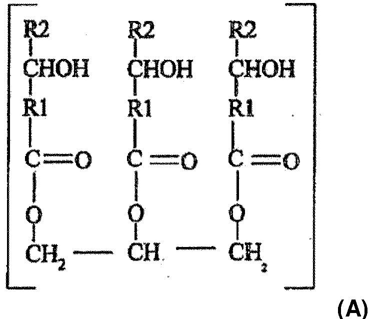 dans laquelle
R₁ représente un groupe alkylène saturé ou insaturé, linéaire ou ramifié, comprenant par exemple de 1 à 18 atomes de carbone, et
R₂ représente un groupe alkyle saturé ou insaturé, linéaire ou ramifié, comprenant par exemple de 1 à 12 atomes de carbone,
et
le diacide saturé est représenté par la formule (B) suivante 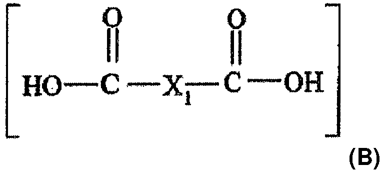
dans laquelle
X₁ est un groupe alkylène linéaire ou ramifié, de préférence un groupe alkylène linéaire -(CH₂)ₓ- où x est un nombre entier compris entre 1 et 30, et de préférence entre 3 et 15.

3. Composition selon la revendication 1 ou 2, dans laquelle l'oligomère (a) d'un triglycéride d'acide gras hydroxylé et d'un diacide saturé est un copolymère d'huile de ricin hydrogénée et d'acide sébacique.

4. Composition selon l'une quelconque des revendications 1 à 3, dans laquelle la quantité d'oligomère(s) (a) d'un triglycéride d'acide gras hydroxylé et d'un diacide saturé est de 0,1 % à 15 % en poids, de préférence de 0,3 % à 10 % en poids, et plus préférablement de 0,5 % à 5 % en poids, par rapport au poids total de la composition.

5. Composition selon l'une quelconque des revendications 1 à 4, dans laquelle la quantité de silicone(s) aminé(s) (b) est de 0,01 % à 15 % en poids, de préférence de 0,05 % à 10 % en poids, et plus préférablement de 0,1 % à 5 % en poids, par rapport au poids total de la composition.

6. Composition selon l'une quelconque des revendications 1 à 5, dans laquelle la composition comprend en outre (c) au moins un silicone autre que le silicone aminé (b).

7. Composition selon l'une quelconque des revendications 1 à 6, dans laquelle le silicone (c) a un degré de polymérisation inférieur à 2000.

8. Composition selon l'une quelconque des revendications 1 à 7, dans laquelle la quantité de silicone(s) (c) est de 0,1 % à 30 % en poids, de préférence de 0,5 % à moins de 25 % en poids, et plus préférablement de 1 % à 20 % en poids, par rapport au poids total de la composition.

9. Composition selon l'une quelconque des revendications 1 à 8, dans laquelle la composition comprend en outre (d) au moins un ester de polyol(s) et de dimère de diacide gras, ou un ester de celui-ci.

10. Composition selon l'une quelconque des revendications 1 à 9, dans laquelle la composition comprend en outre (e) de l'eau.

11. Composition selon l'une quelconque des revendications 1 à 10, dans laquelle la composition comprend en outre (f) au moins un alcool gras.

12. Composition selon l'une quelconque des revendications 1 à 11, dans laquelle la composition comprend en outre (g) au moins un tensioactif cationique.

13. Composition selon l'une quelconque des revendications 1 à 12, dans laquelle la composition est une composition cosmétique, de préférence une composition cosmétique de type rinçage, et plus préférentiellement une composition cosmétique capillaire de type rinçage.

14. Procédé cosmétique pour le soin ou le conditionnement des fibres kératiniques, de préférence des cheveux, comprenant l'étape suivante :
application sur les fibres kératiniques de la composition selon l'une quelconque des revendications 1 à 13.

15. Utilisation de
(a) au moins un oligomère d'un triglycéride d'acide gras hydroxylé et d'un diacide saturé ; et
(b) au moins un silicone aminé,
dans laquelle
le (b) silicone aminé a un degré de polymérisation inférieur à 2000, dans une composition destinée à traiter les fibres kératiniques afin de renforcer ou d'améliorer les effets de conditionnement de la composition pour les fibres kératiniques.
